# EUROPÄISCHE PATENTSCHRIFT

(11) **EP 1 456 663 B1**
(45) Veröffentlichungstag und Bekanntmachung des Hinweises auf die Patenterteilung: **09.05.2007**
(21) Anmeldenummer: 02795260.5
(22) Anmeldetag: 20.12.2002
(51) Int. Cl.: G01N 33/566, C07K 16/28, G01N 33/66, C12Q 1/48

(54) **VERFAHREN ZUR BESTIMMUNG DER RESPONSIVITÄT EINES INDIVIDUUMS FÜR MISTELLEKTIN**
METHOD FOR DETERMINATION OF THE RESPONSIVENESS OF AN INDIVIDUAL TO MISTLETOE LECTIN
PROCEDE POUR DETERMINER LA RESPONSIVITE D'UN INDIVIDU A LA MISTELLECTINE

(30) Priorität: 21.12.2001 EP 01130745
(43) Veröffentlichungstag der Anmeldung: 15.09.2004
(73) Patentinhaber: Viscum AG, 51429 Bergisch-Gladbach (DE)
(72) Erfinder: MÜTHING, Johannes, 33611 Bielefeld (DE); PETER-KATALINIC, Jasna, 14468 Potsdam (DE); LANGER, Martin, 76227 Karlsruhe (DE); MÖCKEL, Babette, 64287 Darmstadt (DE); ECK, Jürgen, 64646 Heppenheim (DE)
(74) Vertreter: Einsel, Martin
(86) Internationale Anmeldenummer: PCT/EP2002/014682
(87) Internationale Veröffentlichungsnummer: WO 2003/054544

(56) Entgegenhaltungen:
- WO-A-02/067850
- US-A- 5 194 385
- GALANINA O E ET AL: "Further refinement of the description of the ligand-binding characteristics for the galactoside-binding mistletoe lectin, a plant agglutinin with immunomodulatory potency." JOURNAL OF MOLECULAR RECOGNITION, Bd. 10, Nr. 3, Mai 1997 (1997-05), Seiten 139-147, XP008020427 ISSN: 0952-3499 in der Anmeldung erwähnt
- WU ALBERT M ET AL: "Interaction of mistletoe toxic lectin-I with sialoglycoproteins." BIOCHEMICAL AND BIOPHYSICAL RESEARCH COMMUNICATIONS, Bd. 214, Nr. 2, 1995, Seiten 396-402, XP002250772 ISSN: 0006-291X in der Anmeldung erwähnt
- SCHWARTZ-ALBIEZ R: "CDw76." JOURNAL OF BIOLOGICAL REGULATORS AND HOMEOSTATIC AGENTS, Bd. 14, Nr. 4, Dezember 2000 (2000-12), Seiten 286-287, XP008020599 ISSN: 0393-974X
- IWAMORI M ET AL: "SELECTIVE TERMINAL ALPHA-2-3 AND ALPHA-2-6 SIALYLATION OF GLYCOSPHINGOLIPIDS WITH LACTO-SERIES TYPE 1 AND 2 CHAINS IN HUMAN MECONIUM" FEBS (FEDERATION OF EUROPEAN BIOCHEMICAL SOCIETIES) LETTERS, Bd. 233, Nr. 1, 1988, Seiten 134-138, XP001164112 ISSN: 0014-5793
- ANDRE SABINE ET AL: "Neoglycoproteins with the synthetic complex biantennary nonasaccharide or its alpha2,3/alpha2,6-sialylated derivatives: Their preparation, assessment of their ligand properties for purified lectins, for tumor cells in vitro, and in tissue sections, and their biodistribution in tumor-bearing mice." BIOCONJUGATE CHEMISTRY, Bd. 8, Nr. 6, November 1997 (1997-11), Seiten 845-855, XP002250773 ISSN: 1043-1802 in der Anmeldung erwähnt
- MÜTHING JOHANNES ET AL: "Preferential binding of the anticancer drug rViscumin (recombinant mistletoe lectin) to terminally alpha2-6-sialylated neolacto-series gangliosides." GLYCOBIOLOGY. ENGLAND AUG 2002, Bd. 12, Nr. 8, August 2002 (2002-08), Seiten 485-497, XP008020013 ISSN: 0959-6658 in der Anmeldung erwähnt
- "alpha2-6-sialylated neolacto-series gangliosides serve as receptors for the anticancer drug rViscumin" EUROPEAN JOURNAL OF CANCER, PERGAMON PRESS, OXFORD, GB, Bd. 38, November 2002 (2002-11), Seiten S107-S108, XP004403804 ISSN: 0959-8049
- VALENTINER URSULA ET AL: "The cytotoxic effect of mistletoe lectins I, II and III on sensitive and multidrug resistant human colon cancer cell lines in vitro." TOXICOLOGY, Bd. 171, Nr. 2-3, 28. Februar 2002 (2002-02-28), Seiten 187-199, XP002250774 ISSN: 0300-483X
- SCHWARTZ-ALBIEZ REINHARD ET AL: "Characterization of sialyltransferase activity of B cell lines expressing alpha-2,6-sialylated surface antigens" & "Fine specificity analysis of B cell workshop antibodies reactive with sialic acid dependent carbohydrate epitopes" TISSUE ANTIGENS, Bd. 42, Nr. 4, 1993, Seite 321, XP002264988 5th International Conference on Human Leukocyte Differentiation Antigens;Boston, Massachusetts, USA; November 3-7, 1993 ISSN: 0001-2815
- DATABASE BIOSIS [Online] BIOSCIENCES INFORMATION SERVICE, PHILADELPHIA, PA, US; Februar 1998 (1998-02), BREEN KIERAN C ET AL: "The generation and characterization of a rat neural cell line overexpressing the alpha2,6(N) sialyltransferase" XP002264920 Database accession no. PREV199800184335 & GLYCOCONJUGATE JOURNAL, Bd. 15, Nr. 2, Februar 1998 (1998-02), Seiten 199-202, ISSN: 0282-0080

### SEQUENCE LISTING

<110> Viscum AG
<120> Verfahren zur Bestimmung der Responsivität eines Individuums für Mistellektin
<130> F 1827 PCT
<160> 6
<170> Patent In version 3.1
<210> 1
   <211> 855
   <212> DNA
   <213> Viscum album
<400> 1
   atgaatgcgg ttatggactc aagaagggca tgggcttcgt gttttttaat gctgggccta      60
   gtttttggtg cgacggtcaa agcggaaacc aaattcagct acgagaggct aagactcaga    120
   gttacgcatc aaaccacggg cgacgaatat ttccggttca tcacgcttct ccgagattat    180
   gtctcaagcg gaagcttttc caatgagata ccactcttgc gtcagtctac gatccccgtc    240
   tccgatgcgc aaagatttgt cttggtggag ctcaccaacc aggggggaga ctcgatcacg     300
   gccgccatcg acgttaccaa tctgtacgtc gtggcttacc aagcaggcga ccaatcctac    360
   tttttgcgcg acgcaccacg cggcgcggaa acgcatctct tcaccggcac cacccgatcc    420
   tctctcccat tcaacggaag ctaccctgat ctggagcgat acgccggaca tagggaccag    480
   atccctctcg gtatagacca actcattcaa tccgtcacgg cgcttcgttt tccgggcggc    540
   agcacgcgta cccaagctcg ttcgatttta atcctcattc agatgatctc cgaggccgcc    600
   agattcaatc ccatcttatg gagggctcgc caatacatta acagtggggc gtcatttctg    660
   ccagacgtgt acatgctgga gctggagacg agttggggcc aacaatccac gcaagtccag    720
   cattcaaccg atggcgtttt taataaccca attcggttgg ctataccccc cggtaacttc    780
   gtgacgttga ccaatgttcg cgacgtgatc gccagcttgg cgatcatgtt gtttgtatgc    840
   ggagagcggc catct                                                      855
<210> 2
   <211> 285
   <212> PRT
   <213> Viscum album
<400> 2
   Met Asn Ala Val Met Asp Ser Arg Arg Ala Trp Ala Ser Cys Phe Leu
   1               5                   10                  15
   Met Leu Gly Leu Val Phe Gly Ala Thr Val Lys Ala Glu Thr Lys Phe
               20                  25                  30
   Ser Tyr Glu Arg Leu Arg Leu Arg Val Thr His Gln Thr Thr Gly Asp
           35                  40                  45
   Glu Tyr Phe Arg Phe Ile Thr Leu Leu Arg Asp Tyr Val Ser Ser Gly
       50                  55                  60
   Ser Phe Ser Asn Glu Ile Pro Leu Leu Arg Gln Ser Thr Ile Pro Val
   65                  70                  75                  80
   Ser Asp Ala Gln Arg Phe Val Leu Val Glu Leu Thr Asn Gln Gly Gly
                   85                  90                  95
   Asp Ser Ile Thr Ala Ala Ile Asp Val Thr Asn Leu Tyr Val Val Ala
               100                 105                 110
   Tyr Gln Ala Gly Asp Gln Ser Tyr Phe Leu Arg Asp Ala Pro Arg Gly
           115                 120                 125
   Ala Glu Thr His Leu Phe Thr Gly Thr Thr Arg Ser Ser Leu Pro Phe
       130                 135                 140
   Asn Gly Ser Tyr Pro Asp Leu Glu Arg Tyr Ala Gly His Arg Asp Gln
   145                 150                 155                 160
   Ile Pro Leu Gly Ile Asp Gln Leu Ile Gln Ser Val Thr Ala Leu Arg
                   165                 170                 175
   Phe Pro Gly Gly Ser Thr Arg Thr Gln Ala Arg Ser Ile Leu Ile Leu
               180                 185                 190
   Ile Gln Met Ile Ser Glu Ala Ala Arg Phe Asn Pro Ile Leu Trp Arg
           195                 200                 205
   Ala Arg Gln Tyr Ile Asn Ser Gly Ala Ser Phe Leu Pro Asp Val Tyr
       210                 215                 220
   Met Leu Glu Leu Glu Thr Ser Trp Gly Gln Gln Ser Thr Gln Val Gln
   225                 230                 235                 240
   His Ser Thr Asp Gly Val Phe Asn Asn Pro Ile Arg Leu Ala Ile Pro
                   245                 250                 255
   Pro Gly Asn Phe Val Thr Leu Thr Asn Val Arg Asp Val Ile Ala Ser
               260                 265                 270
   Leu Ala Ile Met Leu Phe Val Cys Gly Glu Arg Pro Ser
           275                 280                 285
<210> 3
   <211> 789
   <212> DNA
   <213> Viscum album
<400> 3
   gatgatgtta cctgcagtgc ttcggaacct acggtgcgga ttgtgggtcg aaatggcatg      60
   tgcgtggacg tccgagatga cgatttccgc gatggaaatc agatacagtt gtggccctcc     120
   aagtccaaca atgatccgaa tcagttgtgg acgatcaaaa gggatggaac cattcgatcc     180
   aatggcagct gcttgaccac gtatggctat actgctggcg tctatgtgat gatcttcgac     240
   tgtaatactg ctgtgcggga ggccactctt tggcagatat ggggcaatgg gaccatcatc     300
   aatccaagat ccaatctggt tttggcagca tcatctggaa tcaaaggcac tacgcttacg     360
   gtgcaaacac tggattacac gttgggacag ggctggcttg ccggtaatga taccgcccca     420
   cgcgaggtga ccatatatgg gttcagggac ctttgcatgg aatcaaatgg agggagtgtg    480
   tgggtggaga cgtgcgtgag tagccaaaag aaccaaagat gggctttgta cggggatggt    540
   tctatacgcc ccaaacaaaa ccaagaccaa tgcctcacct gtgggagaga ctccgtttca    600
   acagtaatca atatagttag ctgcagcgct ggatcgtctg ggcagcgatg ggtgtttacc    660
   aatgaagggg ccattttgaa tttaaagaat gggttggcca tggatgtggc gcaagcaaat    720
   ccaaagctcc gccgaataat catctatcct gccacaggaa aaccaaatca aatgtggctt    780
   cccgtgcca                                                            789
<210> 4
   <211> 263
   <212> PRT
   <213> Viscum album
<400> 4
   Asp Asp Val Thr Cys Ser Ala Ser Glu Pro Thr Val Arg Ile Val Gly
   1               5                   10                  15
   Arg Asn Gly Met Cys Val Asp Val Arg Asp Asp Asp Phe Arg Asp Gly
               20                  25                  30
   Asn Gln Ile Gln Leu Trp Pro Ser Lys Ser Asn Asn Asp Pro Asn Gln
           35                  40                  45
   Leu Trp Thr Ile Lys Arg Asp Gly Thr Ile Arg Ser Asn Gly Ser Cys
       50                  55                  60
   Leu Thr Thr Tyr Gly Tyr Thr Ala Gly Val Tyr Val Met Ile Phe Asp
   65                  70                  75                  80
   Cys Asn Thr Ala Val Arg Glu Ala Thr Leu Trp Gln Ile Trp Gly Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Gly Gly Ser Val
   145                 150                 155                 160
   Trp Val Glu Thr Cys Val Ser Ser Gln Lys Asn Gln Arg Trp Ala Leu
                   165                 170                 175
   Tyr Gly Asp Gly Ser Ile Arg Pro Lys Gln Asn Gln Asp Gln Cys Leu
               180                 185                 190
   Thr Cys Gly Arg Asp Ser Val Ser Thr Val Ile Asn Ile Val Ser Cys
           195                 200                 205
   Ser Ala Gly Ser Ser Gly Gln Arg Trp Val Phe Thr Asn Glu Gly Ala
       210                 215                 220
   Ile Leu Asn Leu Lys Asn Gly Leu Ala Met Asp Val Ala Gln Ala Asn
   225                 230                 235                 240
   Pro Lys Leu Arg Arg Ile Ile Ile Tyr Pro Ala Thr Gly Lys Pro Asn
                   245                 250                 255
   Gln Met Trp Leu Pro Val Pro
               260
<210> 5
   <211> 1958
   <212> DNA
   <213> Viscum album
<400> 5
   ttttatctcc tgccatcttc catcggggag tcgccgtgac accattcagg aacaatgaat      60
   gcggttatgg actcaagaag ggcatgggct tcgtgttttt taatgctggg cctagttttt     120
   ggtgcgacgg tcaaagcgga aaccaaattc agctacgaga ggctaagact cagagttacg     180
   catcaaacca cgggcgacga atatttccgg ttcatcacgc ttctccgaga ttatgtctca     240
   agcggaagct tttccaatga gataccactc ttgcgtcagt ctacgatccc cgtctccgat     300
   gcgcaaagat ttgtcttggt ggagctcacc aaccaggggg gagactcgat cacggccgcc     360
   atcgacgtta ccaatctgta cgtcgtggct taccaagcag gcgaccaatc ctactttttg    420
   cgcgacgcac cacgcggcgc ggaaacgcat ctcttcaccg gcaccacccg atcctctctc     480
   ccattcaacg gaagctaccc tgatctggag cgatacgccg gacataggga ccagatccct     540
   ctcggtatag accaactcat tcaatccgtc acggcgcttc gttttccggg cggcagcacg     600
   cgtacccaag ctcgttcgat tttaatcctc attcagatga tctccgaggc cgccagattc     660
   aatcccatct tatggagggc tcgccaatac attaacagtg gggcgtcatt tctgccagac     720
   gtgtacatgc tggagctgga gacgagttgg ggccaacaat ccacgcaagt ccagcattca     780
   accgatggcg tttttaataa cccaattcgg ttggctatac cccccggtaa cttcgtgacg     840
   ttgaccaatg ttcgcgacgt gatcgccagc ttggcgatca tgttgtttgt atgcggagag     900
   cggccatctt cctctgaggt gcgctattgg ccgctggtca tacgacccgt gatagccgat     960
   gatgttacct gcagtgcttc ggaacctacg gtgcggattg tgggtcgaaa tggcatgtgc    1020
   gtggacgtcc gagatgacga tttccgcgat ggaaatcaga tacagttgtg gccctccaag    1080
   tccaacaatg atccgaatca gttgtggacg atcaaaaggg atggaaccat tcgatccaat    1140
   ggcagctgct tgaccacgta tggctatact gctggcgtct atgtgatgat cttcgactgt    1200
   aatactgctg tgcgggaggc cactctttgg cagatatggg gcaatgggac catcatcaat    1260
   ccaagatcca atctggtttt ggcagcatca tctggaatca aaggcactac gcttacggtg    1320
   caaacactgg attacacgtt gggacagggc tggcttgccg gtaatgatac cgccccacgc    1380
   gaggtgacca tatatgggtt cagggacctt tgcatggaat caaatggagg gagtgtgtgg    1440
   gtggagacgt gcgtgagtag ccaaaagaac caaagatggg ctttgtacgg ggatggttct    1500
   atacgcccca aacaaaacca agaccaatgc ctcacctgtg ggagagactc cgtttcaaca    1560
   gtaatcaata tagttagctg cagcgctgga tcgtctgggc agcgatgggt gtttaccaat    1620
   gaaggggcca ttttgaattt aaagaatggg ttggccatgg atgtggcgca agcaaatcca    1680
   aagctccgcc gaataatcat ctatcctgcc acaggaaaac caaatcaaat gtggcttccc    1740
   gtgccatgat ttaggttcat ggctcgaaga ttgcttgcat gcgaccatcc tttctatttt    1800
   ctcttttcta ccttttgaaa taatgtctgt gaataatgtg gcacgttgag gcccgccgaa    1860
   agaagcctta gccaccttgt gtttgagaat aaatgagtta atgcaagcaa tcaacttctc    1920
   cttaaaaaaa aaaaaaaaaa aaaaaaaaaa aaaaaaaa                            1958
<210> 6
   <211> 647
   <212> PRT
   <213> Viscum album
<400> 6
   Phe Tyr Leu Leu Pro Ser Ser Ile Gly Glu Ser Pro His His Ser Gly
   1               5                   10                  15
   Thr Met Asn Ala Val Met Asp Ser Arg Arg Ala Trp Ala Ser Cys Phe
               20                  25                  30
   Leu Met Leu Gly Leu Val Phe Gly Ala Thr Val Lys Ala Glu Thr Lys
           35                  40                  45
   Phe Ser Tyr Glu Arg Leu Arg Leu Arg Val Thr His Gln Thr Thr Gly
       50                  55                  60
   Asp Glu Tyr Phe Arg Phe Ile Thr Leu Leu Arg Asp Tyr Val Ser Ser
   65                  70                  75                  80
   Gly Ser Phe Ser Asn Glu Ile Pro Leu Leu Arg Gln Ser Thr Ile Pro
                   85                  90                  95
   Val Ser Asp Ala Gln Arg Phe Val Leu Val Glu Leu Thr Asn Gln Gly
               100                 105                 110
   Gly Asp Ser Ile Thr Ala Ala Ile Asp Val Thr Asn Leu Tyr Val Val
           115                 120                 125
   Ala Tyr Gln Ala Gly Asp Gln Ser Tyr Phe Leu Arg Asp Ala Pro Arg
       130                 135                 140
   Gly Ala Glu Thr His Leu Phe Thr Gly Thr Thr Arg Ser Ser Leu Pro
   145                 150                 155                 160
   Phe Asn Gly Ser Tyr Pro Asp Leu Glu Arg Tyr Ala Gly His Arg Asp
                   165                 170                 175
   Gln Ile Pro Leu Gly Ile Asp Gln Leu Ile Gln Ser Val Thr Ala Leu
               180                 185                 190
   Arg Phe Pro Gly Gly Ser Thr Arg Thr Gln Ala Arg Ser Ile Leu Ile
           195                 200                 205
   Leu Ile Gln Met Ile Ser Glu Ala Ala Arg Phe Asn Pro Ile Leu Trp
       210                 215                 220
   Arg Ala Arg Gln Tyr Ile Asn Ser Gly Ala Ser Phe Leu Pro Asp Val
   225                 230                 235                 240
   Tyr Met Leu Glu Leu Glu Thr Ser Trp Gly Gln Gln Ser Thr Gln Val
                   245                 250                 255
   Gln His Ser Thr Asp Gly Val Phe Asn Asn Pro Ile Arg Leu Ala Ile
               260                 265                 270
   Pro Pro Gly Asn Phe Val Thr Leu Thr Asn Val Arg Asp Val Ile Ala
           275                 280                 285
   Ser Leu Ala Ile Met Leu Phe Val Cys Gly Glu Arg Pro Ser Ser Ser
       290                 295                 300
   Glu Val Arg Tyr Trp Pro Leu Val Ile Arg Pro Val Ile Ala Asp Asp
   305                 310                 315                 320
   Val Thr Cys Ser Ala Ser Glu Pro Thr Val Arg Ile Val Gly Arg Asn
                   325                 330                 335
   Gly Met Cys Val Asp Val Arg Asp Asp Asp Phe Arg Asp Gly Asn Gln
               340                 345                 350
   Ile Gln Leu Trp Pro Ser Lys Ser Asn Asn Asp Pro Asn Gln Leu Trp
           355                 360                 365
   Thr Ile Lys Arg Asp Gly Thr Ile Arg Ser Asn Gly Ser Cys Leu Thr
       370                 375                 380
   Thr Tyr Gly Tyr Thr Ala Gly Val Tyr Val Met Ile Phe Asp Cys Asn
   385                 390                 395                 400
   Thr Ala Val Arg Glu Ala Thr Leu Trp Gln Ile Trp Gly Asn Gly Thr
                   405                 410                 415
   Ile Ile Asn Pro Arg Ser Asn Leu Val Leu Ala Ala Ser Ser Gly Ile
               420                 425                 430
   Lys Gly Thr Thr Leu Thr Val Gln Thr Leu Asp Tyr Thr Leu Gly Gln
           435                 440                 445
   Gly Trp Leu Ala Gly Asn Asp Thr Ala Pro Arg Glu Val Thr Ile Tyr
       450                 455                 460
   Gly Phe Arg Asp Leu Cys Met Glu Ser Asn Gly Gly Ser Val Trp Val
   465                 470                 475                 480
   Glu Thr Cys Val Ser Ser Gln Lys Asn Gln Arg Trp Ala Leu Tyr Gly
                   485                 490                 495
   Asp Gly Ser Ile Arg Pro Lys Gln Asn Gln Asp Gln Cys Leu Thr Cys
               500                 505                 510
   Gly Arg Asp Ser Val Ser Thr Val Ile Asn Ile Val Ser Cys Ser Ala
           515                 520                 525
   Gly Ser Ser Gly Gln Arg Trp Val Phe Thr Asn Glu Gly Ala Ile Leu
       530                 535                 540
   Asn Leu Lys Asn Gly Leu Ala Met Asp Val Ala Gln Ala Asn Pro Lys
   545                 550                 555                 560
   Leu Arg Arg Ile Ile Ile Tyr Pro Ala Thr Gly Lys Pro Asn Gln Met
                   565                 570                 575
   Trp Leu Pro Val Pro Phe Arg Phe Met Ala Arg Arg Leu Leu Ala Cys
               580                 585                 590
   Asp His Pro Phe Tyr Phe Leu Phe Ser Thr Phe Asn Asn Val Cys Glu
           595                 600                 605
   Cys Gly Thr Leu Arg Pro Ala Glu Arg Ser Leu Ser His Leu Val Phe
       610                 615                 620
   Glu Asn Lys Val Asn Ala Ser Asn Gln Leu Leu Leu Lys Lys Lys Lys
   625                 630                 635                 640
   Lys Lys Lys Lys Lys Lys Lys
                   645

## Beschreibung

Die vorliegende Erfindung betrifft ein Verfahren zur Bestimmung der Responsivität eines Individuums auf Mistellektin oder für eine Mistellektin-Einzelkette, wobei die Expression eines spezifischen, membranständigen Rezeptors für Mistellektin charakteristisch für eine entsprechende Responsivität ist.

Verschiedene Dokumente werden im Text dieser Beschreibung zitiert. Der Offenbarungsgehalt der zitierten Dokumente (einschließlich aller Herstellerbeschreibungen, -angaben etc.) ist hiermit per Referenz Teil dieser Beschreibung.

Mistellektin ist ein dem Ricin verwandtes Typ II Ribosomen inaktivierendes Protein (RIP), welches aus zwei Proteinketten aufgebaut ist (Barbieri et al., 1993). Die A-Kette besitzt dabei eine enzymatische rRNA-N-Glykosidase Aktivität, die B-Kette weist eine Carbohydrat-Bindeaktivität auf. Rekombinantes in *E*. *coli* dargestelltes rViscumin ist dem Fachmann bekannt (EP 0 751 221 B1). Insbesondere wird in *E*. *coli* hergestelltes rViscumin als Monosubstanz klinisch entwickelt. rViscumin entspricht in seiner Primärstruktur nicht genau einem der in der Mistel-Pflanze zu findenden ML-I, ML-II oder ML-III. Es ist davon auszugehen, daß es neben der beschriebenen rViscumin-Sequenz noch weitere, durch Punktmutationen in dem ursprünglichen Gen modifizierte Mistellektin-Varianten gibt, die sich leicht in ihrer Primärstruktur unterscheiden können. rViscumin könnte als eine Variante des Ur-Mistellektins gesehen werden und als Mischung aus ML-I, ML-II und ML-III Sequenzen verstanden werden.
Die Aktivitäten beider Ketten des Mistellektins sind für die cytotoxische/cytostatische Aktivität des Proteins notwendig. Dabei kommt dem ersten Schritt, nämlich der Bindung des Moleküls auf der Oberfläche der Zelle, eine entscheidende Bedeutung zu.
Wie dem Ricin werden auch dem Mistellektin (und dem rekombinant in *E*. *coli* hergestellten rViscumin; Eck et al. 1999a, Eck et al., 1999b) Galactose/Lactose Spezifität zugeschrieben (Olsnes et al., 1982; Lee et al., 1992; Gilleron et al., 1998). Die Art der glykosidischen Verknüpfung der endständigen Galactose und des nachfolgenden Zuckers wurde hierbei bisher als unerheblich für die Spezifität des Lektins beschrieben (Lee et al., 1994; Gupta et al., 1996).
Es werden verschiedene Mistellektine (ML-I, -II und -III) in der Literatur beschrieben, die sich in ihrer Carbohydrat-Spezifität unterscheiden (Franz, 1986). Dabei wird diskutiert, daß sich die Spezifität von Galactose/Lactose (ML-I) über eine Mischform aus Galactose/Lactose und N-Acetyl-Galactosamin (ML-II) hin zu einem Mistellektin, welches stärker an N-Acetyl-Galactosamin (ML-III) bindet, verändert. Das rViscumin zeigt eine in verschiedenen ELISA-ähnlichen Verfahren detektierbare Galactose/Lactose Bindeaktivität (Eck et al., 1999b). Andere Carbohydrat-Bindeaktivitäten sind für Mistellektine bisher nur sehr rudimentär und zum Teil auch konträr beschrieben. So beobachteten z.B. Wu et al. (1995a und 1995b), daß die Fähigkeit von ML-I entweder humanes α1-saures-Glykoprotein oder Fetuin zu binden signifikant reduziert ist, wenn die desialylierten Pendants eingesetzt werden. Im Gegensatz dazu fanden diese Autoren eine Erhöhung der Bindung und eine komplette Präzipitation, wenn Siaologlykoprotein aus der Ratte in desialylierter Form in dem Test eingesetzt wurde. Die von Wu et al. gezogenen Schlüsse sind jedoch aufgrund der widersprüchlichen Ergebnisse der eingesetzten Sialinsäure tragenden bzw. desialylierten Proteine nicht interpretierbar. Auch ist festzuhalten, daß die Glykosylierung der Proteine nicht einheitlich ist. Ein Desialylierungsvorgang erfolgt chemisch und variiert nach Herstellerangaben. So kann in einer desialylierten Probe auch noch ein Rest von endständig Sialinsäure tragender Proteine sein, welche die kommunizierten Ergebnisse verfälschen. Trotzdem wurde eine Spezifität des ML-I für Sialinsäure diskutiert, da es den Autoren gelungen ist, die Protein-Mistellektin Wechselwirkung einzudämmen, wenn die Oligosaccharid-Mischung Neu5Acα2-3/Neu5Acα2-6Gal-β1-4Glc als Kompetitor eingesetzt wurde. In der tabellarischen Zusammenfassung der Kompetitionsergebnisse eines Präzipitationsexperiments (Mikroprezipitin Technik) fällt jedoch auf, daß bei gleichem Versuchsaufbau eine Präzipitation von ML-1 durch diverse Glykoproteine (humanes α-1 saures Glykoprotein; Fetuin, Asialo-RSL) sowohl durch Lactose als auch durch das Gemisch Neu5Acα2-3/Neu5Acα2-6Gal-β1-4Glc sowie durch Galβ1-4GlcNAc in etwa gleichen Konzentrationen verhindert werden kann. Eine Spezifität von ML-1 für Sialinsäure ist aus diesen Daten nicht eindeutig abzulesen, nicht zuletzt deshalb, weil sich die Ergebnisse die mit Asialo-RSL und dem endständig Sialinsäure tragenden Fetuin in der Tendenz absolut gleichen.
Etwa zeitgleich konnte von Debray et al. (1994) gezeigt werden, daß die Affinität von an Sepharose 4B immobilisiertem ML-I gegenüber entweder O-3 oder O-6 sialylierter Lactose bzw. Sialo-N-Glykosylpeptiden im Vergleich zu N-Acetyllactosamin Typ Oligosacchariden und Glykopeptiden leicht zugenommen hat. Debray et al. (1994) verwenden hierbei Oligosaccharide und Glykopeptide, die sie teilweise aus humanen Quellen (z.B. Urin, humanes Serumtransferrin, humanes α-1 saures Glykoprotein) isoliert haben. Debray et al. führten Untersuchungen an einer Säule durch, an der sie Mistellektin-I immobilisierten (ML-I-Sepharose). Bei der Einordnung der getesteten Saccharide unterschieden sie drei Fraktionen. Fraktion 1 (FNR) zeigte dabei keine Wechselwirkung mit dem Mistellektin und eluierte in PBS im Elutionsvolumen der Säule. Eine zweite Fraktion (FR) eluierte zeitlich etwas verzögert aber immer noch unter Verwendung von PBS. Saccharide dieser beiden Fraktionen unterscheiden sich entsprechend nur gering in ihrer Kapazität zur Wechselwirkung mit dem auf der Säule immobilisierten Lektin. Die eigentlich bindende Fraktion (FE) konnte erst durch 150 mM Galactose in PBS-Puffer eluiert werden. Die Ergebnisse sind nicht schlüssig. So diskutieren die Autoren eine Retardierung der Elution von FNR zu FR, wenn sie an die endständigen Galactose-Reste eine Sialinsäure α2-6 verknüpfen (vergleiche Saccharide 16 (FR) und 17 (FNR)). Allerdings führt eine doppelte Sialinsäure-Markierung wie sie in Saccharid 15 (FR) zu sehen ist nicht dazu, daß die Substanz stärker an das ML-I an der Säule bindet. Es fällt jedoch dem Fachmann schwer, explizit über Retardierungen von FNR zu FR zu diskutieren. So ist es sehr wahrscheinlich, daß die leichte Retardierung (FR) durch unspezifische Wechselwirkungen (z.B. hydrophobe Wechselwirkungen) mit dem an die Säule gebundenen Protein herrührt und nichts mit der Spezifität des Mistellektins zu tun hat. Nur zwei der getesteten Strukturen eluieren nach Spülen der Säule mit PBS Puffer + 150 mM NaCl + 150mM Galactose (FE). Diese beiden Strukturen wurden aus bovinem Thyroglobulin bzw. Turteltauben Ovomucoid isoliert. Im Gegensatz zu allen anderen Strukturen, die von den Autoren verwendet wurden und vornehmlich die Struktur Gal(β1-4)GlcNAc- enthielten, sind diese Strukturen mit endständig zwei Galactose Resten ausgestattet, welche entweder α1-4 oder α1-3 mit der zweiten Galactose verknüpft sind.
Lee et al. (1994) beschrieben die Relevanz des zweiten Zuckerrestes bei der Erkennung des ML-I. Sie unterteilen die Erkennung von Zuckerstrukturen durch ML-I in vier Gruppen und konnten zeigen, daß GlcNAc Strukturen an der zweiten Position die Erkennung der Zucker stark beeinträchtigen. Die stärkste Affinität des ML-I fanden sie zu einem Saccharid mit den endständigen Zuckerresten β-D-Gal-(1-2)-β-D-Gal-. Auch β1-3 verknüpfte Galactosen zeigten ähnlich gute Spezifitäten. β1-4 verknüpfte Galactosen wurden von Lee et al. (1994) nicht getestet, aus den Arbeiten von Debray et al. (1994) läßt sich jedoch schließen, daß auch diese eine hohe Spezifität zu ML-I haben müßten.
Bei der Bewertung der Arbeiten von Debray et al. (1994) muß jedoch beachtet werden, daß die Untersuchungen mit Mistellektin durchgeführt wurde, welches auf einer Säule immobilisiert wurde. Die beobachteten Wechselwirkungen der Saccharide mit dem immobilisierten Lektin stellen ein unphysiologisches, experimentelles System dar. Ein Rückschluß auf die Wechselwirkung von gelöstem Mistellektin, bzw. rViscumin in Lösung mit einem Rezeptor ist folglich nicht direkt möglich.
Die hier dargestellten Daten von Lee et al. (1994), Debray et al. (1994) und Wu et al. (1995) zur Spezifität von Mistellektin sind in sich widersprüchlich und können nicht als schlüssiger Beweis für eine Spezifität des Mistellektins gegenüber Neu5Ac gesehen werden. Insbesondere ist problematisch, daß die Daten nicht unter definierten Versuchsbedingungen durchgeführt wurden. Im Wesentlichen bezieht sich dies auf die Qualität der eingesetzten Proteine, die technisch bedingt, nie eine homogene Carbohydrat Struktur aufweisen.
Auf der Basis der Beobachtungen von Lee et al. (1992) entwickelten Galanina et al. (1997) ein experimentelles System, in dem Mistellektin an strukturell definierte Neoglykokonjugate gekoppelt wurde. In diesem System wurde die kompetitive Potenz synthetischer Oligosaccharide zur Aufhebung der Bindung von Mistellektin untersucht. Die mit Hilfe dieses Systems gewonnenen Ergebnisse sind jedoch ebenfalls heterogen. Erwartungsgemäß wurde eine kompetitive Potenz von Lactose gezeigt. Die Kompetition mit N-Acetyllactosamin führt zu ähnlichen Ergebnissen. Darüber hinaus wurden auch natürlich vorkommende lsomere der Sialyllactose untersucht. Das α2-3 sialylierte Isomer besaß hierbei eine höhere kompetitive Aktivität als das α2-6 sialylierte Isomer, die jedoch deutlich unterhalb jener von Lactose oder N-Acetyllactosamin lag. Weiter führten die Autoren auf, daß N-Acetylneuraminsäure allein keine inhibitorische Aktivität hat.
Bei der Analyse der auf Kompetitionsstudien beruhenden Arbeiten fällt auf, daß die untersuchten, natürlich vorkommenden Glykoproteine nur lösliche Proteine umfaßten. Membranständige Glykoproteine wurden in den beschriebenen Experimenten nicht untersucht.

Mistellektin, aber auch z.B. andere Lactose/Galactose spezifische Proteine wie Ricin oder Galectin werden als Asialofetuin-bindende Proteine beschrieben. Diese Eigenschaft läßt sich auch für eine Quantifizierung zunutze machen (Vang et al., 1986). Gupta et al. (1996) konnten die Wechselwirkung der Proteine Ricin, Galectin oder Mistellektin (hier als *Viscum album*-Agglutinin bezeichnet) etwas genauer beschreiben. Sie fanden heraus, daß alle drei Proteine definierte Komplexe mit Asialofetuin ausbilden. Der oder die Rezeptoren, die für die Bindung des Mistellektin oder des Ricin an die Zielzelle verantwortlich sind, sind noch nicht bekannt.
1982 wurde der Rezeptor für das Cholera Toxin auf Balb/c 3T3 Zellen identifiziert (Critchley et al., 1982). Es handelt sich hier um das Gangliosid GM1. Ausgehend von dieser Arbeit wurde versucht, auch die Rezeptoren anderer Toxine/Lektine in diesem Umfeld zu suchen. Dabei konnte für Ricin und Erdnuß Agglutinin gezeigt werden, daß es sich bei den Rezeptoren auf humanen Lymphocyten um Glykoproteine handelt, während Ricinus Agglutinin und Sojabohnen Agglutinin zu etwa gleichen Teilen an Glykoproteine und Ganglioside binden (Turpin et al., 1984). In Untersuchungen mit Modellmembranen, in die das Monosialo-Gangliosid GM1 (mit terminaler Galactose) eingebaut war, wurden nur sehr unspezifische Wechselwirkungen mit den beiden Proteinen Ricin und Mistellektin beobachtet, die keine klare Unterscheidung der Permeabilität der Membranen in Abhängigkeit des zugesetzten Typ II RIPs ermöglichte (Pohl et al., 1998a). Darüber hinaus beobachteten Pohl et al. (1998b), daß sowohl Ricin als auch Mistellektin in der Lage seien, Vesikel-Vesikel Fusionen zu induzieren. Das Modell der Fusions-Induktion hat aber keine entscheidende Relevanz für die Aufnahme von Misetellektin oder Ricin in vivo, da Membranfusionen nicht für die Aufnahme dieser Proteine durch eine Zielzelle verantwortlich gemacht werden. Bereits 1990 zeigten Tonevitsky et al., daß das Gangliosid GM1 nicht als der Rezeptor gesehen werden kann. Utsumi et al. (1987) berichteten über eine Bindung von Ricin an GM1 enthaltende Liposomen, die durch die Zugabe des Kompetitors Lactose nicht beobachtet werden konnte.
Samal et al. (1995) beobachteten, daß Mistellektin ebenso wie Ricin Blutplättchen aggregiert. Mistellektin aggregiert nach dieser Untersuchung jedoch keine aus Blutplättchen isolierte Liposomen, die von Ricin aggregiert werden. Ein potentieller Rezeptor für diese Lektine wurde von den Autoren nicht diskutiert.

1994 wurde beobachtet, daß die veränderte Oberflächenstruktur von entarteten Zellen ausgenutzt werden kann(Gottstein et al., 1994; Usui & Hakomori, 1994). Beispielsweise wurden Immuntoxine für eine Therapie vorgeschlagen, die aus monoklonalen Antikörpern gegen spezifische, möglichst nur auf den entarteten Zellen befindliche Glykolipid- bzw. Glykoprotein-Strukturen und der toxischen Komponente des Ricins (Ricin A-Kette) bestehen (Gottstein et al., 1994; Usui & Hakomori, 1994). Dieses Modell verdeutlicht die Relevanz eines Zell-spezifischen Vehikels, welches einen Transport eines Toxins in eine Zielzelle ermöglicht.
Im Fall von Ricin erfüllt die carbohydratbindende B-Kette die Anforderungen eines solchen Vehikels nicht, da für Ricin eine Bindung an einen zwar nicht eindeutig identifizierten, jedoch ubiquitär vorkommenden Rezeptor beschrieben wurde. Diese Beobachtung erklärt die beschriebenen Nebenwirkungen bei einer Therapie von Patienten mit Ricin.
Da Mistellektin im Gegensatz zu Ricin an einen Rezeptor zu binden scheint, der nicht ubiquitär vorkommt, wurde die B-Kette als mögliches Vehikel für einen Transport von fusionierten Toxinen vorgeschlagen. Entsprechende Fusionsproteine der B-Kette des rViscumins mit zytotoxischen Verbindungen wurden in der EP-Anmeldung EP 1012256 A1 beschrieben. Für einen effizienten und zielgerichteten Einsatz entsprechender Therapeutika wäre es deshalb wünschenswert den Rezeptor zu identifizieren, an den die B-Kette des Mistellektins bzw. des rekombinant hergestellten rViscumins bindet.

Die Verwendung von Mistelextrakten (Extrakte von Viscum album) als Heilmittel sind schon seit Jahrhunderten bekannt. Als wirksame Bestandteile dieser Extrakte wurden hierbei als Lektine bezeichnete Inhaltsstoffe identifiziert. Bei diesen Lektinen handelt es sich um Eiweißstoffe, die sehr spezifische Kohlenhydratstrukturen auch in lipid- oder proteingebundener Form erkennen und an diese binden. Mistellektin, das als Ribosomen inaktivierendes Protein der Klasse II charakterisiert wurde, wirkt pharmakologisch nur durch das Zusammenspiel seiner beiden Untereinheiten. Die B-Kette des Mistellektins, die Sequenzmotive mit spezifischen kohlenhydratbindenden Eigenschaften besitzt, ist hierbei für den Transport des Proteins in die Zielzelle verantwortlich. In der Zielzelle blockiert dann die A-Untereinheit durch ihre enzymatische rRNA-N-Glycosidase-Aktivität den ribosomalen Stoffwechsel der Zelle und löst auf diese Weise einen programmierten Zelltod (Apoptose) in dieser aus.
In dem europäischen Patent EP 0 602 686 B1 wurde die Wirkungsweise der Mistelpflanze und der daraus gewonnenen Extrakte zur Behandlung von Krankheiten beschrieben. Seit Anfang dieses Jahrhunderts werden Mistelpräparate zur Krebstherapie mit unterschiedlichem Erfolg angewandt (Bocci, 1993; Gabius et al., 1994; Gabius & Gabius, 1994; Ganguly & Das, 1994). Hajto et al. (1989, 1990) konnten zeigen, daß die therapeutischen Effekte insbesondere durch sogenannte Mistellektine (Viscumine, Viscum album Agglutinine, VAA) vermittelt werden. Es wird neben einer zytotoxischen Wirkung heute insbesondere eine (unspezifische) Immunstimulation diskutiert, deren positive Effekte zur begleitenden Therapie und zur Nachsorge von Tumorpatienten ausgenutzt werden. Eine Steigerung der Lebensqualität bei solchen Patienten wird möglicherweise durch die Ausschüttung körpereigener Endorphine vermittelt (Heiny und Beuth, 1994).

Zahlreiche Untersuchungen in vitro (Hajto et al., 1990; Männel et al., 1991; Beuth et al., 1993) und in vivo (Hajto, 1986; Hajto et al., 1989, Beuth et al., 1991; Beuth et al., 1992), sowie klinische Studien (Beuth et al., 1992) belegen die durch Mistellektin vermittelte erhöhte Freisetzung von inflammmatorischen Zytokinen (TNF-α, IL-1, IL-6) sowie eine Aktivierung von zellulären Komponenten des Immunsystems (TH-Zellen, NK-Zellen).
Als aktives Prinzip der Mistelextrakte wird heute ein 60kDa Mistellektin-Protein angesehen, das auf biochemischem Weg aus Extrakten gewonnen werden kann (Franz et al., 1977; Gabius et al., 1992). Das ML-Protein besteht aus zwei kovalent S-S verbrückten Untereinheiten, dessen A-Kette für eine enzymatische Inaktivierung von Ribosomen (Endo et al., 1988) und dessen B-Kette für die Carbohydratbindung verantwortlich ist. Die biologische Aktivität wird korreliert mit dem Erhalt der Lektinaktivität der B-Kette (Hajto et al., 1990).

Der vorliegenden Erfindung liegt das technische Problem zugrunde, daß bisher keine gezielte Voraussage getroffen werden kann, ob eine Therapie, die eine Gabe von rViscumin oder ähnlicher Verbindungen einschließt, zur Behandlung einer Erkrankung oder eines Leides eines Individuums geeignet ist.

Dieses technische Problem wird durch die in den Ansprüchen charakterisierten Ausführungsformen gelöst.

Folglich betrifft die vorliegende Erfindung ein in vitro Verfahren zur Bestimmung der Responsivität eines Individuums für Mistellektin oder für (eine) Mistellektin-Einzelkette(n), umfassend den Schritt des spezifischen quantitativen und/oder qualitativen Nachweises eines membranständigen Rezeptors, wobei der Rezeptor durch eine endständige N-Acetyl-Neuraminsäure (Neu5Ac), die über eine glykosidische α2-6-Verknüpfung mit einer Galactose (Gal) verbunden ist, gekennzeichnet ist.

Erfindungsgemäß wird daher vorzugsweise ein in vitro Verfahren zur Bestimmung der Responsivität eines Individuums für Mistellektin oder für (eine) Mistellektin-Einzelkette beschrieben, wobei die Responsivität durch die spezifische Bindung der carbohydratbindenden Untereinheit des Mistellektins an einen membranständigen Rezeptor vermittelt wird und der Rezeptor durch eine endständige N-Acetyl-Neuraminsäure (Neu5Ac) gekennzeichnet ist, die über eine glykosidische α2-6-Verknüpfung mit einer Galactose (Gal) verbunden ist. Dabei umfasst die Bestimmung den spezifischen quantitativen und/oder qualitativen Nachweis der genannten spezifischen Glykosylierung.

Der Begriff "Mistellektin" umfasst sowohl die hierin beschriebenen und dem Fachmann bekannten natürlichen als auch rekombinanten Mistellektine, wobei die oben erwähnte Mistellektin-Einzelkette vorzugsweise die Mistellektin B-Kette oder (ein) Fragment(e) hiervon umfasst.

Der Begriff "Responsivität" definiert im Zusammenhang mit der Erfindung das Auslösen einer die Heilung einer Krankheit oder eines Leidens begünstigenden Reaktion einer einzelnen Zelle, einer Zellpopulation, eines Zellverbands, eines Gewebes, eines Organs oder eines Organismus. Weiterhin umfaßt der Begriff "Responsivität" auch die Empfindlichkeit einer einzelnen Zelle, einer Zellpopulation, eines Zellverbands, eines Gewebes, eines Organs oder eines Organismus in präventiven Einsätzen des rViscumins oder ähnlicher Verbindungen. Folglich ist eine Responsivität entsprechender Zielzellen mit einem positiven Therapie-Effekt oder aber einem präventiven Effekt verbunden. Vorzugsweise umfaßt die "Responsivität" die Empfindlichkeit einer humanen Zelle, Zellpopulation, eines Zellverbands, Gewebes, Organs oder eines menschlichen Organismus.

Der Begriff "spezifische Bindung" kann beispielsweise mit einem "Schlüssel-Schloß-Prinzip" charakterisiert werden. Der Ligand (Mistellektin) und das Zielmolekül (membranständiger Rezeptor) besitzen Strukturen oder Motive, die spezifisch zueinander passen. Ein Beispiel hierfür sind eine antigene Determinante (Epitop), die mit der Antigen-Bindungsstelle eines Antikörpers wechselwirkt. Entsprechend steht spezifische Bindung im Gegensatz zu einer universelleren, unspezifischen Bindung. Durch die Kenntnis der Struktur eines der spezifisch aneinander bindenden Interaktionspartner lassen sich Rückschlüsse auf mögliche bevorzugte Strukturen bzw. auf spezielle Strukturelemente eines geeigneten damit interagierenden Partners ziehen. Die Erfindung verwendet den spezifischen Interaktionspartner für Mistellektin, insbesondere rViscumin.

Der Begriff "carbohydratbindende Untereinheit des Mistellektins" beschreibt die Sequenzmotive der B-Kette des Mistellektins, die spezifisch an den membranständigen Rezeptor des Mistellektins binden. Diese Motive wurden von Langer et al. (2000) beschrieben. Wie auch die carbohydratbindende Untereinheit des Ricins ist die B-Kette des Mistellektins aus 2 Domänen aufgebaut. Diese werden jeweils wiederum in 3 Subdomänen unterteilt. Die Domänen werden als 1 und 2, die Subdomänen werden als α, β und γ bezeichnet. Für Ricin ist beschrieben, daß sich jede Subdomäne von einer vermutlich bakteriellen Carbohydrat-Bindestruktur ableitet (Rutenber et al. 1987). Aufgrund der hohen strukturellen Identität der B-Kette des Ricins und des Mistellektins (62% Identität und 70% Homologie nach Eck et al., 1999) scheint diese Beobachtung auch auf das Mistellektin übertragbar zu sein. Die unterschiedliche Spezifität der carbohydratbindenden Untereinheit des Ricins und des Mistellektins ist nach Langer et al. (2000) auf die Unterschiede in den Subdomänen zurückzuführen. Dies sind im besonderen in der 1α-Subdömäne die Reste D23 und W38, in 1β die Reste Y68, Y70, Y75 und F79 und in Subdomäne 2γ die Reste D235, Y249; zur Numerierung siehe Eck et al. (1999a).

Der Begriff "membranständiger Rezeptor" definiert im Zusammenhang mit der Erfindung eine membrangebundene Struktur, die durch eine endständige N-Acetyl-Neuraminsäure (Neu5Ac) gekennzeichnet ist. Diese ist wiederum über eine glykosidische α2-6-Verknüpfung mit einer Galactose (Gal) verbunden. Beispiele für entsprechende membrangebundene Strukturen sind Proteine oder Peptide, die mit der Membran einer Zelle verankert sind. Diese Definition umfaßt sowohl transmembrane, als auch membranassoziierte Proteine und Peptide. Ebenfalls sind Lipide, die entweder selbst Teil der Membran sind oder aber an diese assoziiert sind, Beispiele für solche Strukturen. Die offenbarte membrangebundene Struktur kann sowohl Teil eines Glykoproteins (glykosyliertes Protein), als auch eines Glykolipids sein.

Der Begriff "Membran" umfaßt in diesem Zusammenhang alle membranösen, zellulären Lipiddoppelschichten. Entsprechend sind sowohl Membranen des Endoplasmatischen Reticulums (ER), des Golgi, der Zellkernhülle, von Vesikeln und Vakuolen, als auch der äußeren Zellmembran explizit umfaßt.

Im Stand der Technik werden Proteine beschrieben, die mit einer endständige N-Acetyl-Neuraminsäure (Neu5Ac) glykosyliert sind, die wiederum über eine glykosidische α2-6-Verknüpfung mit einer Galactose (Gal) verbunden sind. Diese glykosylierten Proteine sind jedoch lösliche Proteine/Serumproteine (siehe z.B. Hanasaki et al. 1995), die weder für die pharmakologischen Effekte noch die Wirkungsweise von Mistellektin relevant sind. Im Zusammenhang mit dieser Erfindung wird erstmals eine glykosylierte, membrangebundene Struktur offenbart.

Die Identifikation endständiger N-Acetyl-Neuraminsäure (Neu5Ac), die über eine glykosidische α2-6-Verknüpfung mit einer Galactose (Gal) verbunden ist, als spezifische Zielstruktur der carbohydratbindenden Untereinheit des Mistellektins erlaubt es einem Fachmann allein oder in Kombination mit der Lehre aus Langer et al. (2000) beispielsweise die carbohydratbindende Untereinheit des Ricins durch punktspezifische Mutagenese zu verändern. Ein entsprechend mutiertes Peptid/Protein könnte dann nicht mehr an Ricin-spezifische Zielstrukturen, sondern statt dessen an Mistellektin-spezifische Zielstrukturen binden.

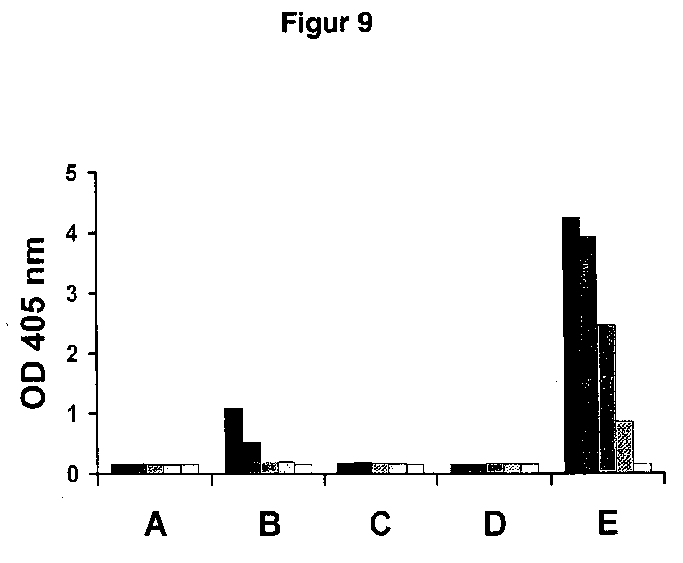

Beispiele für einen quantitativen und/oder qualitativen Nachweis der genannten Glykosylierung sind dem Fachmann bekannt und unter anderem im angefügten Beispiel 7 und in der dieses Beispiel illustrierenden Figur 9 beschrieben. Solche Verfahren umfassen zum Beispiel modifizierte Western-Blot Analysen, wie in den Beispielen gezeigt. Ebenfalls umfassen solche Verfahren, Techniken wie z.B. den Radioimmuno-Assay (RIA), Sandwich (immunometrische assay) und Western-Blot Assay, IRMA (Immune Radioimmunometric Assay), EIA (Enzyme Immuno Assay), ELISA (Enzyme Linked Immuno Sorbant Assay), FIA (Fluorescent Immuno Assay), CLIA (Chemioluminescent Immune Assay), Agglutinations-Assay und durchflußzytometrische Verfahren.

Das erfindungsgemäße Verfahren ermöglicht, inter alia, eine Prognose für die Wirksamkeit einer Mistellektin-Therapie. Dies schließt ein, daß eine Voraussage gemacht werden kann, ob eine Therapie mit Mistellektin, vorzugsweise mit rViscumin, zur Behandlung einer Erkrankung in bestimmten Zellen, einer bestimmten Zellpopulation oder einem bestimmten Gewebe, Organ oder Organismus grundsätzlich Aussicht auf einen Therapieerfolg hat. Wird beispielsweise mit Hilfe des erfindungsgemäßen Verfahrens die oben genannte Glykosylierung auf einer Tumorzelle identifiziert, so kann rViscumin an die Zelle binden, von dieser aufgenommen werden und in dieser zytotoxisch wirken. Darüber hinaus ermöglicht das erfindungsgemäße Verfahren eine Prognose für die Wirksamkeit einer Mistellektin-Therapie in einem Individuum. In einem Patientenkollektiv können folglich individuelle Responder (Patienten, die auf die Therapie ansprechen) von Nicht-Respondern unterschieden werden. Dieses Prognoseverfahren erfüllt damit eine ähnliche Aufgabe wie dem Fachmann bekannte Untersuchungen, die vor einer Therapie von Brustkrebspatienten mit Herceptin durchgeführt werden. Bevor eine Therapie mit dem Antikörper-Präparat Herceptin verabreicht werden darf, muß untersucht werden, ob ein Patient den EGF-Rezeptor Her-2 besitzt. Von der FDA (Food and Drug Administration der USA) sind in diesem Zusammenhang insbesondere zwei Testverfahren zugelassen (immunhistochemische Färbung (IHC) und Fluoreszenz *in situ*-Hybridisierung (FISH)). Siehe hierzu u.a. Thomson et al. (2001). Analog können solche Verfahren und weitere, hierin beschriebene Techniken eingesetzt werden, um, gemäß der erfinderischen Lehre, individuelle Responder für eine Mistellektin-Therapie, insbesondere eine Therapie mit rViscumin, zu erkennen und/oder zu bestimmen.

Für das erfindungsgemäße Verfahren können Proben von Flüssigkeiten, insbesondere Körperflüssigkeiten, Zellen, Zellpopulationen oder Geweben verwendet werden. Diese Proben können aus Blutproben oder anderen Proben von Körperflüssigkeiten stammen, aber auch Gewebeproben, einzelne Zellen oder dessiminierte Tumorzellen sein. Beispiele für solche Proben und deren Entnahme werden in dieser Anmeldung im Zusammenhang mit weiteren Ausführungsformen näher beschrieben.

Das erfindungsgemäße Verfahren ermöglicht ebenfalls eine frühzeitige Erkennung von Respondern in einem Patientenkollektiv. Auf diese Weise ist es möglich in einem einfach durchzuführenden Test ohne eine größere Verzögerung vor einem Therapiebeginn den Erfolg dieser Therapie abzuschätzen. Dieses Charakteristikum des erfindungsgemäßen Verfahrens ist vor allem aus krankheitsökonomischen Gesichtspunkten relevant. Es wird eine, gegebenenfalls kostenintensive, für den individuellen Patienten aber ineffektive Therapie vermieden. Auf diese Weise können auch bei einer effektiven Therapie in Kauf genommenen Nebenwirkungen bei diesen individuellen Patienten, bei denen ein Therapie-Erfolg mit rViscumin zweifelhaft erscheint, vermieden werden.

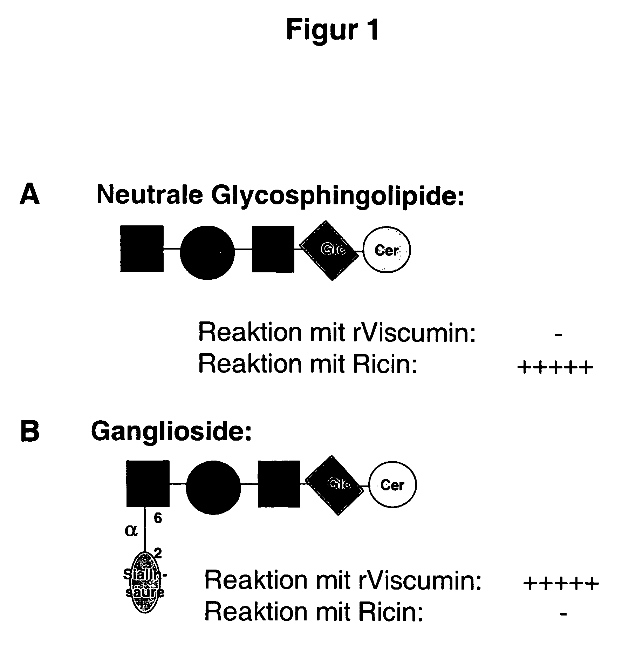

In den der Erfindung zu Grunde liegenden und in den Beispielen dargestellten Untersuchungen konnte zweifelsfrei gezeigt werden, daß Mistellektin und Ricin an Glycosphingolipide (GSL) binden. Zum ersten Mal konnte hier ein quantitativer und qualitativer Unterschied zwischen der Bindespezifität von Mistellektin (insbesondere rViscumin) und Ricin dokumentiert werden. Die von den beiden Proteinen erkannten Carbohydratstrukturen sind schematisch in Figur 1 dargestellt.

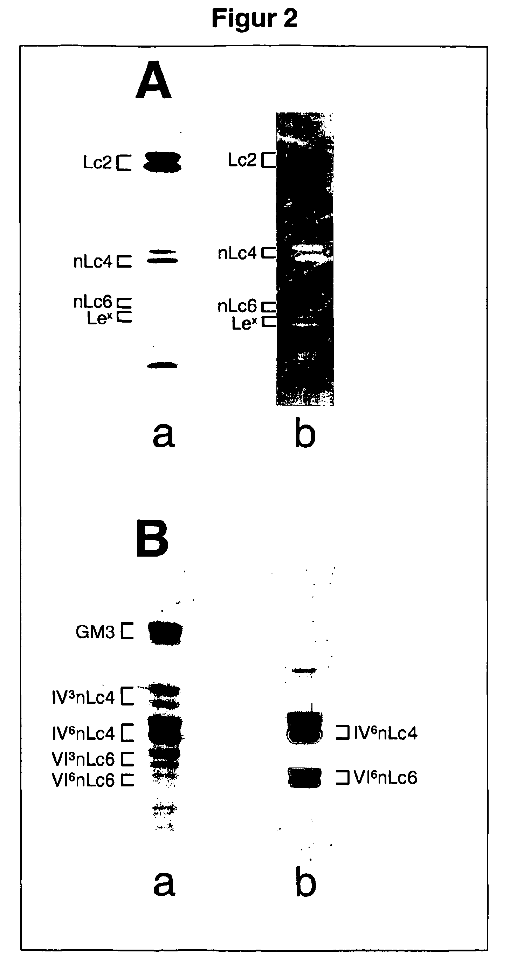

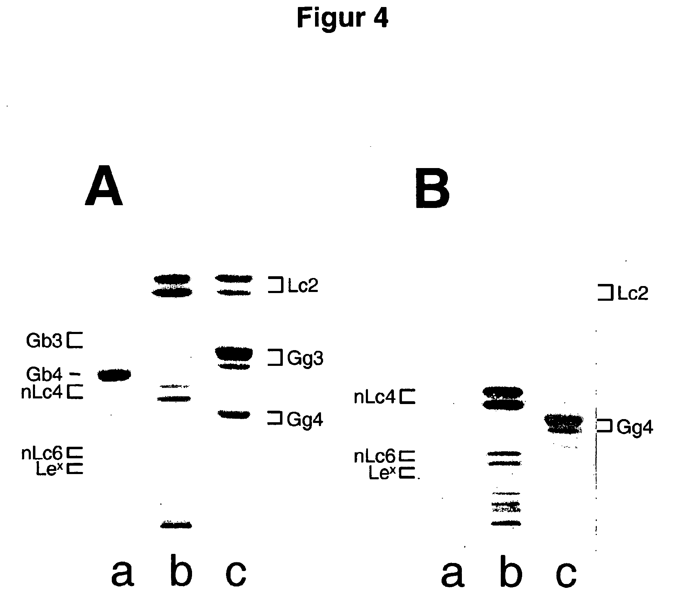

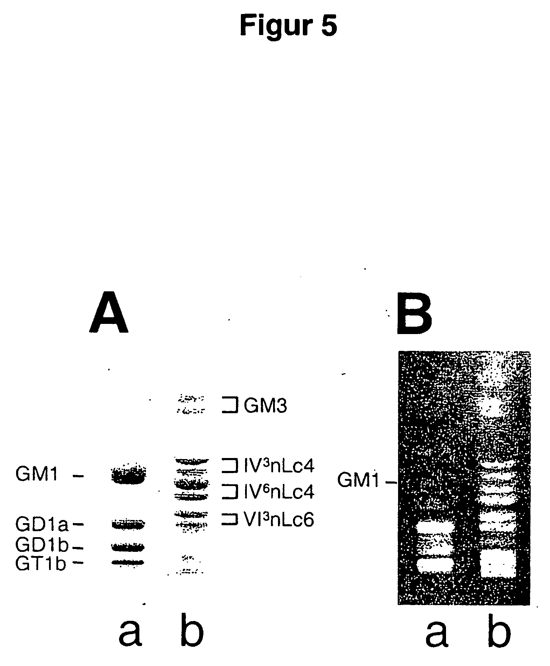

Wie in den illustrierten Beispielen dargestellt, konnte in spezifischen Tests gezeigt werden, daß die primäre Spezifität von Mistellektin (insbesondere rViscumin) nicht eine endständige Galactose ist. In Figuren 2 bis 5 und 7 ist ein modifizierter Western Blot/immunologischer Nachweis von gebundenem Ricin bzw. rViscumin auf einer DC-Platte (TLC Assay) gezeigt, auf der die neutralen und sauren GSL aufgrund ihrer unterschiedlichen Laufeigenschaften in verschiedenen Fließmitteln vorher getrennt wurden. Während Ricin eine klare Spezifität für neutrale, GSL mit endständiger Galactose (Lc2, nLc4, nLc6, Gg4) zeigt (Tabelle 1; Fig. 4B Spur b), zeigt rViscumin überraschender Weise gegen Galβ1-4Glcβ1-1Cer (Cer steht hier für Ceramid) nur eine äußerst schwache Bindung (Tabelle 1; Fig. 2 A, Spur b, "negative-stain" nach ü.N. Inkubation).
Es wurde überraschender Weise gezeigt, daß dies jedoch nicht für eine Gruppe der Ganglioside gilt, an denen endständig eine N-Acetyl-Neuraminsäure (Neu5Ac) sitzt. Diese wird von rViscumin sehr gut und von Ricin überhaupt nicht erkannt (siehe Tabelle 1; Figur 2B, Spur b; Figur 5 B, Spur b "negative stains"). Weiterhin wurde überraschender Weise gefunden, daß es für die Erkennung der Rezeptorstruktur durch rViscumin entscheidend ist, ob die N-Acetyl-Neuraminsäure mit der terminalen Galactose des Neutralzuckergrundgerüstes α2-6 (wird erkannt) oder α2-3 (wird nicht erkannt) verknüpft ist (siehe angefügte Beispiele und Figuren).
Diese erfinderische, spezifische Erkennung von N-Acetyl-Neuraminsäure in Neu5Ac-α2-6-Gal-Konfiguration erinnert an die Spezifität in der Epitoperkennung von monoklonalen Antikörpern und wurde bisher für Mistellektin noch nicht beschrieben. Auch die absolut unterschiedliche Spezifität von Ricin und rViscumin war überraschend und für den Fachmann nicht vorhersagbar. Die unterschiedlichen Erkennungsmotive der beiden getesteten Typ II Ribosomen inaktivierenden Proteine sind in der Tabelle 1 zusammengefaßt. Vom Stand der Technik könnte geschlossen werden, daß sowohl Ricin, als auch Mistellektin (insbesondere rViscumin), wenn überhaupt, eher an die neutralen GSL binden, die als endständigen Zuckerrest eine Galactose besitzen.

In einer bevorzugten Ausführungsform des Verfahres umfaßt der Rezeptor Ganglioside, die nach der α2-6-sialylierten Galactose mindestens ein N-Acetyl-Glucosamin (GIcNAc) besitzen, d.h. Ganglioside, die nach der α2-6-gebundenen N-Acetyl-Neuraminsäure an Galactose mindestens ein N-Acetyl-Glucosamin (GIcNAc) umfassen. Wie unten aufgeführt, kann jedoch das anschließende N-Acetyl-Glucosamin (GlcNAc) sowohl direkt als auch indirekt an die Neu5Acα2-6-Gal-Struktur anschließen. Es ist daher im Zusammenhang mit dieser Erfindung möglich, dass das erfinderische Verfahren auf der (Struktur-) Erkennung eines Gangliosidrezeptors beruht, der die Struktur Neu5Acα2-6-Gal-Y-[GlcNAc]ₓ-Cer umfasst. Y kann z.B. eine weitere Galactose (Gal) umfassen.

Der Begriff "Gangliosid" definiert im Zusammenhang mit der Erfindung saure, Sialinsäure enthaltende Ceramidoligosaccharide. Die Kohlenhydrateinheiten sind über eine glycosidische Bindung mit der C1-OH-Gruppe von N-Acylsphingosine (= Ceramid) verknüpft. Ganglioside können sowohl langkettig (z.B. IV³nLc4, VI³nLc6, etc.) wie auch verzweigt sein (GM1 oder GM2). (Voet und Voet (1992), insbesondere Figur auf Seite 271 dieser Referenz).

In einer darüber hinaus bevorzugten Ausführungsform des Verfahrens umfaßt der Rezeptor Ganglioside mit der Struktur Neu5Acα2-6-Gal-[Gal/GlcNAc]ₓ-Cer, wobei Cer ein Ceramid. Besonders bevorzugt ist in dem hier beschriebenen Verfahren jedoch Neu5Acα2-6-[Galβ1-4GlcNAcβ1-3]ₓGalβ1-4Glcβ1-1Cer.
Gängige Verfahren zur strukturellen Charakterisierung von Gangliosiden lassen gegenwärtig nur eine exakte Analyse von Gangliosiden zu, bei denen x einen Wert ≤ 6 hat. Die carbohydratbindende Untereinheit des Mistellektins erkennt die endständigen Zuckerstrukturen. Entsprechend ist der Wert der Variablen x grundsätzlich unerheblich für die spezifische Bindung. Vorzugsweise hat die Variable einen Wert von maximal 10. Darüber hinaus bevorzugt hat die Variable einen Wert von ≤ 6, weiter bevorzugt einen Wert von 5, 4, 3, 2 oder 1. Weiter bevorzugt ist, daß bei verzweigten Gangliosiden der Wert von x in einzelnen oder in allen Ketten unterschiedlich ist.

Ebenfalls bevorzugt umfaßt der Rezeptor in einer Ausführungsform des Verfahrens Ganglioside der Struktur Neu5Acα2-6-[Galβ1-4GlcNAcβ1-3]ₓGalβ1-4Glcβ1-1Cer, besonders bevorzugt ist Neu5Acα2-6Galβ1-4GlcNAcβ1-3Galβ1-4Glcβ1-1Cer oder ein Gangliosid Neu5Acα2-6Galβ1-4GlcNAcβ1-3Galβ1-4GlcNAcβ1-3Galβ1-4Glcβ1-1 Cer.
Auch durch diese bevorzugte Ausführungsform sind lineare und verzweigte Ganglioside umfaßt.

Entsprechend einer weiteren bevorzugten Ausführungsform ist der Rezeptor zellmembranständig.

Diese Ausführungsform des erfindungsgemäßen Verfahrens beschreibt die Analyse von membranständigen Rezeptoren, die Teil der äußeren Zellmembran sind oder an diese gebunden sind. Insbesondere umfaßt diese Ausführungsform Rezeptoren, die Glykoproteine oder Glykolipide sind.

In einer weiteren, bevorzugten Ausführungsform ist das Mistellektin ein rekombinantes Mistellektin/rViscumin.
Wie oben beschrieben werden verschiedene natürlich vorkommende Mistellektine (ML-I, -II und -III) in der Literatur unterschieden. Diese unterscheiden sich in ihrer Carbohydrat-Spezifität (Franz, 1986). Dabei wird diskutiert, daß sich die Spezifität von Galactose/Lactose (ML-I) über eine Mischform aus Galactose/Lactose und N-Acetyl-Galactosamin (ML-II) hin zu einem Mistellektin, welches stärker an N-Acetyl-Galactosamin (ML-III) bindet, verändert. Rekombinantes in *E. coli* dargestelltes Mistellektin/rViscumin ist dem Fachmann bekannt (EP 0 751 221 B1, Eck et al. 1999a und 1999b). rViscumin entspricht in seiner Primärstruktur nicht genau einem der in der Mistel-Pflanze zu findenden ML-I, ML-II oder ML-III. Es ist davon auszugehen, daß es neben der beschriebenen rViscumin-Sequenz noch weitere, durch Punktmutationen in dem ursprünglichen Gen modifizierte Mistellektin-Varianten gibt, die sich leicht in ihrer Primärstruktur unterscheiden können. rViscumin könnte als eine Variante des Ur-Mistellektins gesehen werden und als Mischung aus ML-I, ML-II und ML-III Sequenzen verstanden werden.
Darüber hinaus sind durch diese Ausführungsform rekombinante Fusionsproteine auf der Basis des rViscumins umfaßt, wie diese in der EP-Anmeldung EP A2 1012256 beschrieben werden.

Weiter bevorzugt umfaßt das rekombinante Mistellektin in einer Ausführungsform der Erfindung eine Aminosäuresequenz, die durch ein Polynucleotid wie in SEQ ID Nr: 1, SEQ ID Nr: 3 oder SEQ ID Nr: 5 dargestellt, codiert wird.

In einer darüber hinaus bevorzugten Ausführungsform ist vorzugsweise auch rekombinantes Mistellektin umfaßt, daß durch ein oder mehrere Polynucleotide codiert wird.

Ebenfalls umfaßt das rekombinante Mistellektin in einer bevorzugten Ausführungsform des erfindungsgemäßen Verfahrens ein Polypeptid mit einer Aminosäuresequenz wie in SEQ ID Nr: 2, SEQ ID Nr: 4 oder SEQ ID Nr: 6 dargestellt oder ein funktionelles Fragment hiervon.

Der Begriff "funktionelles Fragment" definiert im Zusammenhang mit dieser Erfindung Fragmente der genannten Polypeptide, welche die gleiche biologische Funktion besitzen, wie die mit Aminosäuresequenz (SEQ ID) dargestellten Polypeptide. Die Funktion von Mistellektin, insbesondere rViscumin, und der bekannten Untereinheiten des Mistellektins ist hierin bereits beschrieben. Die "Funktion" umfaßt auch die hier offenbarte spezifische Bindungseigenschaft des Mistellektins, insbesondere der B-Kette des Mistellektins, für den beschriebenen Mistellektin-Rezeptor. Somit sind auch Fragmente der Mistellektin-B-Kette umfaßt, insbesondere jene Fragmente, die eine spezifische Interaktion mit dem beschriebenen Mistellektin-Rezeptor vermitteln können.
Der Begriff "gleiche biologische Funktion" beschreibt in diesem Zusammenhang beispielsweise, daß Fragmente oder Derivate der Polypeptide die gleichen Signale in einer Zelle induzieren, wie die genannten Peptide. Beispiele für Fragmente sind Peptiddomänen mit definierten Funktionen oder bestimmte prosthetische Gruppen. Die "gleiche biologische Funktion" umfassen auch die Cytotoxizität, Immunstimmulation (sowohl des nativen, als auch des adaptiven Immunsysterns), Stimulation der Freisetzung von Zytokinen, Antigenität, die Induktion der Expression oder die Aktivierung von Oberflächenmarkern (z.B. CD56 auf NK-Zellen), die Induktion von Apoptose oder Endorphin-Stimulation
In einer darüber hinaus bevorzugten Ausführungsform ist auch rekombinantes Mistellektin umfaßt, das durch ein oder mehrere Polynucleotide codiert wird, die für ein Polypeptid mit einer Aminosäuresequenz wie in SEQ ID Nr: 2, SEQ ID Nr: 4 oder SEQ ID Nr: 6 dargestellt oder ein funktionelles Fragment davon codieren, deren Sequenz jedoch unter Berücksichtigung des genetischen Codes degeneriert ist.

Der hier erstmalig beschriebene Mistellektin-Rezeptor spielt auch in zellulären Tests eine Rolle, wie in den Beispielen dargestellt. Es konnte gezeigt werden, daß Zellen auf Behandlung mit rViscumin unterschiedlich reagieren. Insbesondere zeigen Tumorzellen oder Zellen, die von Tumoren abgeleitet sind, spezifische, in den Beispielen beschriebene biochemische Reaktionen auf die Behandlung mit rViscumin.
Durch die in den Beispielen dargestellten Experimente konnte ebenfalls die Spezifität von rViscumin für den hier offenbarten Rezeptor zweifelsfrei identifiziert werden. Darüber hinaus wurde eindeutig gezeigt, daß rViscumin im Vergleich zu Ricin eine quantitativ andere Carbohydrat-Spezifität besitzt.
In den der Erfindung zugrundeliegenden Untersuchungen, die in den angefügten Beispielen beschrieben sind, wurde gezeigt, daß Tumorzellen bzw. Tumorzellinien den oben beschriebenen spezifischen Rezeptor für Mistellektin, insbesondere rViscumin, exprimieren.

Sialyltransferasen sind in eukaryontischen Zellen für die terminale Glykosylierung von glykosylierten Strukturen verantwortlich. Die Aktivität dieser Enzyme wurde im Golgi-Apparat lokalisiert (im Trans-Golgi-Kompartiment). Die Enzyme katalysieren den Transfer von Sialinsäureresten. Die Enzymaktivität solcher Sialyltransferasen katalysiert beispielsweise in entarteten Zellen (Krebszellen) eine spezifische Glykosylierung von Peptiden und/oder Lipiden. Eine Präsenz solcher Sialyltransferasen in Zell- oder Gewebeproben kann als ein Hinweis auf eine Entartung von Zellen gewertet werden. Dies gilt um so mehr für den Nachweis einer enzymatischen Aktivität dieser Sialyltransferasen in einer Probe.
Molekularbiologische und proteinbiochemische Verfahren zur quantitativen und/oder qualitativen Bestimmung von Sialyltransferase(n) sind dem Fachmann aus der Literatur bekannt (siehe u.a. Mülhardt (2000) und Rehm (2000)). Molekularbiologische Methoden sind beispielsweise RT-PCR Techniken, RNAse Protektions Assays, Northern oder Southern Blot-Analyse. Proteinbiochemische Methoden sind beispielsweise Verfahren zum Nachweis einer Transferaseaktivität, aber auch Verfahren wie Western Blot-Analyse oder andere Techniken die unter dem Begriff Proteom-Analyse zusammengefaßt werden können.

Weiterhin kann die quantitative Bestimmung der Sialyltransferase von Einzelzellen, z.B. dissiminierte Tumorzellen umfassen. Einzelzellanalysen sind dem Fachmann bekannt und beschrieben in EP A1 11009938 und Klein (1999).
Ein Beispiel für eine entsprechende Sialyltransferase ist die β-Galactosid α-2,6-Sialyltransferase E.C. 2.4.99.1 (α2-6STN). α2-6STN ist ein 47 kDa Transmembranprotein. Hepatocyten sezernieren auch eine 41 kDa Form dieses Enzyms. Lösliches α2-6STN ist ein Serum-Glykoprotein, welches der Gruppe der "acute phase reactants" zugeordnet wird und in pathologischen Prozessen eine Rolle spielt (verstärkte Aktivität in vielen Karzinomen, z.B. Colon-Karzinom und Cervical-Karzinom). Die Aminosäuresequenzen der bekannten Formen des Enzyms und die dafür codierenden Nukleotidsequenzen sind dem Fachmann bekannt (siehe Accession Nrs L29554 (*Rattus norvegicus),* X75558 *(Gallus gallus*), NM_003032 (*Homo sapiens*) und NM_009175 *(Mus musculus*)).

Die hier dargestellten, erfindungsgemäßen Verfahren können unter Verwendung eines spezifischen Detektionsreagenz durchgeführt werden. Solche spezifischen Detektionsreagenzien sind fähig, mit dem hier beschriebenen Mistellektin-Rezeptor zu interagieren, bzw. zu binden. Diese Interaktion oder Bindung kann direkt oder indirekt sein, sollte jedoch spezifisch für den hierin beschriebenen Rezeptor sein. Besonders bevorzugte Reagenzien sind hierbei spezifische Antikörper, Antikörperfragmente oder Antikörperderivate, Aptamere, carbohydratbindende Moleküle (z.B. Peptide und/oder Proteine), wobei das Reagenz fähig sein muß, den hierin beschriebenen Mistellektin-Rezeptor oder seine spezifischen Bestandteile (z.B. die hierin definierte endständige N-Acetyl-Neuraminsäure, die über eine glykosidische alpha2-6 Verknüpfung mit einer Galactose verbunden ist) zu erkennen und/oder zu binden. Vorzugsweise sind diese Detektionsreagenzien markiert, wobei die Markierung radioaktive Substanzen, Fluoreszenzfarbstoffe, Biotin-(Strept)avidin, Luziferasen, CAT, beta-Galaktosidase, Alkalische Phosphatase(n), Peroxidase(n), weitere enzymatische Markierungen, Digitonin, Farbstoffe im allgemeinen, etc, umfassen kann. Das erfindungsgemäße Verfahren kann jedoch auch mittels indirektem Nachweis des hierin beschriebenen Rezeptors durchgeführt werden. Nachweise des Rezeptors sind in den Beispielen illustriert und können, wie oben erwähnt, auch Verfahren wie RIA, ELISA, CLIA, FIA, ELLA, TLC-Tests, histologische Verfahren, direkte und indirekte (immun)-Fluoreszenzverfahren, oder Durchflußverfahren (z.B. FACS-Analysen, Durchflußzytometrie, BlAcore) umfassen.

In einer alternativen Ausführungsform betrifft die Erfindung eine diagnostische Zusammensetzung, umfassend eine Substanz, die spezifisch einen oben definierten Rezeptor erkennt oder bindet, bevorzugt ausgewählt aus der Gruppe von Antikörpern, Antikörperderivaten, Antikörperfragmenten, Aptameren, wobei die Substanz detektierbar markiert ist oder der Antikörper ein monoklonaler Antiköper ist, der aus einer Hybridomzellinie gewonnen werden kann, die am 20. Dezember 2002 bei der DSMZ Braunschweig unter Zugriffsnummer DSM ACC2580 hinterlegt wurde.

Verfahren zur Herstellung von Antikörpern (polyclonal oder monoclonal), die eine spezifische Zielstruktur erkennen oder binden sind dem Fachmann bekannt.Die Herstellung monoklonaler Antikörper gegen die Ganglioside GD1 und GD2, die bei neurologischen Erkrankungen eine Rolle spielen, wurde beschrieben von Magnani et al. (1982), Tur et al. (2001) und Pan et al. (2001). Der Nachweis der Spezifität solcher Antikörper kann u.a. durch Overlay-Assay, die auf einer lmmunfärbung nach Dünnschichtchromatographie beruhen (Müthing und Mühlradt (1988), Müthing und Kemminer (1996), Müthing (1998)). Dieses Verfahren kann gegebenenfalls auch mit ELISA kombiniert werden.

Die in dem hier beschriebenen Verfahren zu verwendenden Antikörper sind bevorzugterweise monoklonale Antikörper (oder Fragmente oder Derivate hiervon) die spezifisch an die hier offenbarte, membrangebundene Rezeptorstruktur binden. In den Beispielen wird dargelegt, wie solche Antikörper gewonnen werden können. Zum Beispiel sind die in den Beispielen beschriebenen Antikörper mit den Bezeichnungen 59.33.3, 59.33.5 und 59.33.6 gegen die hier beschriebenen α2-6 sialylierte Neolacto-Typ Ganglioside, d.h. den hier beschriebenen Rezeptor für (rekombinantes) Mistellektin gerichtet.

Die Antikörper können, inter alia, Antikörper von lgG, lgA, lgD, lgM-Typ sein. Ein besonders bevorzugter Antikörper kann aus einem Hybridoma/einer Hybridomzellinie gewonnen werden, die unter der Bezeichnung 59.33.3 am 20. Dezember 2002 bei der "Deutschen Sammlung von Mikroorganismen und Zellkulturen GmbH" (DSMZ) in Braunschweig unter der Zugriffsnummer DSM ACC2580 gemäß Budapester Vertrag hinterlegt wurde.
Es ist auch vorgesehen, dass die hier beschriebenen diagnostischen als auch pharmazeutischen Zusammensetzungen (siehe unten) Antikörper umfassen, die von den hier beschriebenen Antikörpern durch Modifikationen dieser gewonnen werden. So können durch dem Fachmann bekannte Techniken die CDR (komplementaritätsbestimmende Regionen; die drei komplementaritätsbestimmenden Regionen CDR1, CDR2 und CDR3 sind Schleifen am Ende einer V-Domäne von Antikörpern oder T-Zell-Rezeptoren. Sie treten mit einem Antigen oder einem Peptid: MHC-Komplex in direkten Kontakt) der hier beschriebenen Antikörper isoliert werden und diese CDR in Antikörper anderer Struktur eingebunden werden. Daher ist es z.B. möglich, die CDR aus des 59.33.3 Antikörpers (ein IgM) zu isolieren und diese CDR in andere Immunglobuline, z.B. in ein IgG, bevorzugt in ein IgG1-Gerüst, zu integrieren. Diese Verfahren (z.B. "CDR-grafting") sind dem Fachmann wohl bekannt. Daher umfassen die hier beschriebenen Antikörper auch chimäre Antikörper, humanisierte Antikörper oder auch einzelkettige Antikörper ("single chain antibodies") oder einzelkettige Antikörperfragmente wie scFv-Konstrukte. Jedoch sind solche Antikörpermodifikationen nicht beschränkt auf Modifikationen der hierin detailliert beschriebenen Antikörper 59.33.3, 59.33.5 oder 59.33.6. Zu verwendete Antikörper können alle Antikörper/Antikörpermoleküle, Antikörperfragmente oder Antikörperderivate sein, die spezifisch an den hierin beschriebenen Rezeptor für (rekombinantes) Mistellektin ((r)Viscumin) binden.

Antikörper-Fragmente, wie Fv, F(ab')₂ und Fab, können durch Spaltung des intakten Proteins (Antikörpers) hergestellt werden, z.B. durch Protease oder durch chemische Spaltung. Es kann aber auch ein verkürztes Gen entworfen werden. Beispielsweise umfasst ein chimäres Gen, das einen Teil des F(ab')₂-Fragments codiert, DNA-Sequenzen, die die CH1-Domäne und Scharnier-Region der H-Kette codieren, gefolgt von einem translationalen Stopp-Codon, wodurch ein verkürztes Molekül erhalten wird.
Der Begriff "Antikörperderivate" beschreibt modifizierte Formen der vorher genannten Antikörper. Diese Modifikationen umfassen chemische, insbesondere biochemische, vorzugsweise proteinbiochemische Modifikationen der Antikörper oder deren Fragmente. Das gemeinsame, die Antikörper, Antikörperfragmente und deren Derivate charakterisierende Merkmal ist hierbei die Erkennung und/oder das Binden an eine spezifischen Zielstruktur. Diese Zielstruktur ist erfindungsgemäß der hierin beschriebene, membrangebundene Rezeptor für Mistellektin.
Aptamere und deren spezifische Eigenschaften sind u.a. von Hermann und Patel (2000) zusammenfassend beschrieben worden. Verfahren zur Isolation von Aptameren sind dem Fachmann ebenfalls aus dem Stand der Technik bekannt.
Der Begriff "Carbohydrat-bindende Peptide" wird hierin definiert wie in der Ausführungsform der Erfindung, die eine Verwendung zur Herstellung eines Arzneimittels betrifft.

In einer darüber hinaus bevorzugten Ausführungsform der diagnostischen Zusammensetzung ist die Substanz detektierbar markiert.
Beispiele für detektierbare Markierungen von Substanzen sind dem Fachmann bekannt. Dies sind u.a. radioaktive, enzymatische oder fluoreszierende Markierungen. Ebenfalls ist die Biotinylierung und eine Detektion mit Hilfe von Avidin oder Streptavidin ein Beispiel für eine entsprechend bevorzugte Ausführungsform.

Eine Ausführungsform der Erfindung umfaßt die Verwendung der erfinderischen diagnostischen Zusammensetzung zur Analyse, ob einzelne Zellen, eine Zellpopulation oder ein Zellverband, Zellen in einem Gewebe, Organ oder Organismus einen funktionellen Rezeptor für Mistellektin besitzen.

In einer weiteren alternativen Ausführungsform betrifft die Erfindung auch die Verwendung einer oben definierten Substanz zur Herstellung eines Diagnostikums zum Nachweis eines funktionellen Mistellektin-Rezeptors.

Weiter bevorzugt ist die erfindungsgemäße Verwendung des Diagnostikums zur Detektion des hierin beschriebenen und in den Beispielen untersuchten Rezeptors auf Zellen.
Wie in den Beispielen dargestellt, zeichnen sich Tumorzellen, die endständige N-Acetyl-Neuraminsäuren (Neu5Ac), die über eine glykosidische α2-6-Verknüpfung mit Galactose (Gal) verbunden sind, exprimieren als besondere Responder auf Mistellektin, insbesondere rekombinant hergestelltes Mistellektin (rViscumin) aus.

In einer bevorzugten Ausführungsform sind die untersuchten Zellen Tumorzellen.
Weiter bevorzugt sind diese Tumorzellen tierische Zellen, vorzugsweise Tumorzellen aus Säugetieren, darüber hinaus bevorzugt Tumorzellen aus Menschen. Darüber hinaus bevorzugt sind diese Zellen leukämische Zellen, kleinzellige und nicht-kleinzellige Lungen-Karzinome, Colon-Karzinome, ZNS-Karzinome, Melanoma, Ovarial-Karzinome, Nieren-Karzinome, Prostata-Karzinome, Mamma-Karzinome, Blasen-Karzinome, Magen-Karzinome, Pancreas-Karzinome oder Hoden-Karzinome.

In einer weiter bevorzugten Ausführungsform der Erfindung stammen die Zellen aus Biopsiematerial.
Verfahren zur Gewinnung von Biopsiematerial sind dem Fachmann bekannt. Durch diese Beschreibung sind unterschiedliche Entnahmearten umfaßt, bei denen Zellen einem Probanden oder Patienten entnommen werden. Bevorzugt sind die Zellen lebende Zellen. Dies sind beispielsweise Feinnadelbiopsien (Feinnadelaspirate), Stanzbiopsien, die Entnahme solider Gewebestücke (z.B. durch chirurgische Verfahren) oder Proben ganzer Organe (z.B. Nebennieren). Die verschiedenen Entnahmetechniken sind z.B. beschrieben in: Kremer et al (1999), Pichlmayr und Löhlein (1991), Niethard und Pfeil (1997), Malte (1998) und Bonk.

In einer ebenfalls bevorzugten Ausführungsform werden die Zellen aus Blutproben isoliert.
Neben einer Isolation von Zellen aus Blutproben betreffen ebenfalls bevorzugte Ausführungsformen der Erfindung die Isolation von Zellen aus Pleuraergüssen, Ascitesproben, Spülflüssigkeiten, Urinproben, Spermaproben oder Proben von Spinal- und Zerebralflüssigkeiten.

Zum Nachweis der Responsivität von z.B. Krebspatienten auf rViscumin wird vor einer geplanten Therapie oder während des Therapieverlaufes Patientengewebe entnommen. Es ist auch möglich auf gesammelte Proben/Konserven (verschiedene)r Patienten zurückgreifen (die in nach Indikationen geordneten Paraffin-Block Banken in vielen Kliniken bevorratet werden), um eine statistisch abgesicherte Aussage z.B. zu einer spezifischen Indikation machen zu können.
Diese Gewebe werden zum Beispiel gefriergetrocknet, in Formalin konserviert oder über Cryokonservierung (gezieltes Einfrieren, z.B. nach Perfusion mit einer cytoprotektiven Lösung,z.B. Zuckerlösung) erhalten und anschließend nach dem Fachmann vertrauten Verfahren weiterverarbeitet, z.B. in Paraffinblöcken konserviert. Ausgehend von den Proben werden für eine mikroskopische Untersuchung geeignete Schnitte hergestellt. Diese Schnitte werden anschliessend mittels dem Fachmann vertrauten Verfahren (z.B. der LSAP Methode einem ELISA-ähnlich Verfahren oder unter Verwendung der ENVISION Methode der Firma DAKO in Hamburg) auf das Vorhandensein der spezifischen Antigene, i.e des hierin definierten Rezeptors für (rekombinantes) Mistellektin untersucht. Hierzu können zum Beispiel die Patientenproben unter Verwendung des hierin beschrieben mAk 59.33.3 oder der beiden anderen, spezifisch das Epitop "CD75s" des hierin beschriebenen Rezeptors erkennenden mAks 59.33.5 oder 59.33.6 verwendet. Gebundene Antikörper, die beipielsweise in einer Konzentration von 500 ng/ml bis 10 µg/ml eingesetzt werden können, werden dann mit sekundären Antikörpern oder anderen Nachweismethoden detektiert. Zum Beispiel kann ein anti-Maus IgM Antikörper, an welchen mit Alkalischer Phosphatase versehenes Polydextran gekoppelt ist, verwendet werden. Ein Vergleich von gesundem zu entartetem Gewebe ist so ebenso möglich wie eine Unterscheidung, ob bei einem Patienten oder bei einer spezifischen Tumor-Indikation eine Behandlung mit rViscumin erfolgversprechend ist. Weiterhin kann nachgewiesen werden, ob eine Behandlung mit (rekombinantem) Mistellektin ((r)Viscumin) erfolgreich ist, d.h. es kann ein therapeutischer Verlauf verfolgt werden.

Weiterhin ermöglicht die hier beschriebene Erfindung andere diagnostische Verfahren, wie, inter alia, die Untersuchung von Feinnadel-Biopsiematerial und Xenograft-Zelllinien oder anderer Zelllinen.
Die Zellen können zum Beispiel in 96-Loch-Platten fixiert werden und in einem ELISA-ähnlichen Verfahren wie unter Beispiel 8 beschrieben auf das Vorhandensein von membrangebundenen/membranbeständigen CD75s-Motiven (rViscumin-Bindemotiven, i.e. der hierin beschriebenen, membrangebundenen Rezeptorstruktur) untersucht werden. Ein anderes Verfahren, was zur Prüfung herangezogen werden kann, ist die FACS-Analyse. Dort können aus einem Pool von Zellen (insbesondere bei Feinnadel-Biopsien interessant) solche herausgelesen werden, die ein bestimmtes Oberflächenantigen tragen. Dabei werden zum Beispiel die Zellen zunächst unter Verwendung von spezifischen Antikörpern, oder Antikörperderivaten, wie zum Beispiel der hierin beschriebenen mAks 59.33.3 59.33.5 oder 59.33.6 verwendet. Gebundene Antikörper, die vorzugsweise aber nicht limitierend in einer Konzentration von 500 ng/ml bis 10 µg/ml eingesetzt werden können, werden dann mit einem sekundären Antikörper, z.B. einem anti-Maus IgM Antikörper, welcher mit FITC oder anderweitig (fluoreszenz)markiert ist, in einen automatischen Cell Sorter z.B. von Becton Dickinson gegeben. Um eine spezifische Zellpopulation heraus zu filtern, kann man bei diesem Verfahren auch ein zweites auf den Zielzellen vorhandenes Epitop mit einem anders als der anti-Maus IgM Antikörper (fluoreszenz-)markierten - Antikörper markieren und dadurch spezifisch nur die in diesem Fall doppelt markierten Zellen herauslesen. Dadurch sind weitere Möglichkeiten gegeben, eine Prädiktivität eines Patienten auf eine rViscumin-Behandlung zu erreichen. Zusätzlich erhält man die Information, wieviel Prozent einer entsprechenden Zellpopulation (doppelt gefärbte Zellen vs. nur mit dem zweiten Antikörper angefärbte Zellen) das gewünschte CD75s Epitop tragen. Aus einer solchen Analyse läßt sich abschätzen bzw. herauslesen ob eine "Minimale Resterkrankung" ("minimal residual disease") vorliegt. Zusätzlich lassen sich so auch Kombinationstherapien mit anderen Cytostatika zusammen mit rViscumin *in vitro* testen und für eine Anwendung am Patienten vorbereiten.

Im diagnostischen Verfahren wie hier beschrieben ist jedoch auch die Analyse von lysierten Membranstrukturen umfasst. **E**s ist, inter alia, möglich, z.B. frisches, nicht fixiertes Biopsiematerial mit Detergenzien (z.B. Triton X-100®, Triton X-112®, Tween 80®, Tween 20®, Octylglycosid, etc.) zu behandeln und das Lysat, z.B. über Westernblotting oder in Form von ELISA/RIA-Tests zu analysieren. D.h. die Detergenzlysate können auf ursprüngliche Anwesenheit des hierin beschriebenen, membrangebundenen Rezeptors für (rekombinantes) Mistellektin ((r)Viscumin) getestet werden. Dem Fachmann stehen ausreichende Verfahren zur entsprechenden Analyse zur Verfügung.

Ein spezifisch an einen oben definierten Rezeptor bindenden oder diesen erkennenden Substanz kann zur Herstellung eines Arzneimittels für die Behandlung von proliferativen Erkrankungen (z.B. Krebs), virale Erkrankungen (z.B. Herpes, HIV), Autoimmunerkrankungen oder neuronalen Erkrankungen wobei die rezeptorbindende oder rezeptorerkennende Substanz ausgewählt ist aus der Gruppe von Antikörpern, Antikörperderivaten, Antikörperfragmenten, Aptameren, niedermolekularen Substanzen und Carbohydrat-bindenden Peptiden verwendet werden. Bevorzugt ist die rezeptorbindende oder rezeptorerkennende Substanz hierbei fähig, eine Bindung der B-Kette von Mistellektin und/oder rViscumin an diesen Rezeptor zu hemmen oder abzuschwächen.
Der Begriff "hemmen" beschreibt im Zusammenhang mit der Erfindung sowohl eine vollständige als auch eine teilweise Inhibition. Somit umfaßt der Begriff eine vollständige Blockade der Bindung von Mistellektin und/oder rViscumin an die als spezifischen Rezeptor identifizierte Carbohydrat-Stuktur. Ebenso ist hiermit die Fähigkeit umfaßt, die Bindung von Mistellektin und/oder rViscumin an den Rezeptor abzuschwächen.
Die oben beschriebene, zur Herstellung eines Arzneimittels verwendete Substanz ist nicht Mistellektin und insbesondere nicht die B-Kette des Mistellektins.
Diese Verwendung betrifft darüber hinaus vorzugsweise die Formulierung von Arzneimitteln, gegebenenfalls in Kombination mit einem "pharmakologisch verträglichen Träger" und/oder Verdünnungsmittel. Beispiele für besonders geeignete pharmakologisch verträgliche Träger sind dem Fachmann bekannt und umfassen gepufferte Kochsalzlösungen, Wasser, Emulsionen wie z.B. ÖI/Wasser-Emulsionen, verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 0,001 und 1000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 10 ng- und 10 mg-Einheiten pro Tag bzw. pro Applikationsintervall befinden. Wird die Zusammensetzung intravenös verabreicht sollte sich die Dosis in einem Bereich zwischen 1 ng- und 0,1 mg-Einheiten pro Kilogramm Körpergewicht pro Minute befinden.
Die Zusammensetzung kann lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die erfindungsgemäße Zusammensetzung kann außerdem Konservierungsmittel und andere Zusätze umfassen, wie z.B. antimikrobielle Verbindungen, Antioxidantien, Komplexbildner und inerte Gase. Des weiteren können, abhängig von der beabsichtigten Verwendung, Verbindungen wie z.B. Interleukine, Wachstumsfaktoren, Differenzierungsfaktoren, Interferone, chemotaktische Proteine oder ein unspezifisches immunmodulatorisches Agens enthalten sein. Ebenso bevorzugt können Zytostatika, Antibiotika und Kombinationen davon enthalten sein.
Hergestellte Arzneimittel oder Medizinprodukte werden entweder zur Vorsorge oder Behandlung eines oben genannten Leidens oder einer Erkrankung verwendet.

Die Herstellung und Verfahren Isolation von Antikörpern, Antikörperfragmenten, Antikörperderivaten und Aptameren sind dem Fachmann, wie oben beschrieben, aus dem Stand der Technik bekannt und wurde oben bereits näher beschrieben.
Erfindungsgemäß sind Substanzen, die eine B-Kette des Mistellektins oder rViscumins oder Derivate davon entsprechen durch die Gruppe der Lektine und Carbohydrat-bindenden Peptide nicht umfaßt. Jedoch umfaßt diese Ausführungsform, inter alia, andere Lektine, die durch biochemische und/oder molekularbiologische Modifikationen (z.B. gerichtete Mutagenese) in ihren Bindungseigenschaften den Bindungseigenschaften der B-Kette für den hierin beschriebenen Rezeptor angepaßt wurden.

Darüber hinaus ist die rezeptorbindende und/oder rezeptorerkennende Substanz in einer weiter bevorzugten Ausführungsform mit einer radioaktiven, zytotoxisch oder zytostatisch wirkenden, Verbindung verknüpft.
Die Art der Verknüpfung der Substanz mit einer radioaktiven, zytotoxisch oder zytostatisch wirkenden Verbindung ist in dieser Ausführungsform abhängig von der Substanz und der Verbindung. Vorzugsweise sind beide beispielsweise Peptide oder Proteine. In diesem Fall erfolgt die Verknüpfung vorzugsweise über eine oder mehrere peptidische Bindungen und/oder Disulfid-Brücken.
Beispiele für Radioimmun-Substanzen sind monoklonale Antikörper (mAb) oder Cytokine, die mit radioaktiven Substanzen markiert sind. Ebenso betrifft eine bevorzugte Ausführungsform die Verknüpfung entsprechender Substanzen mit Lektinen, Toxinen oder Toxoide, vorzugsweise aus Bakterien (z.B. Tetanus-Toxoid, Tetanus-Toxin, Diphtherie-Toxin, Cholera-Toxin, Pseudomonas-Exotoxin, Pseudomonas-Toxoid, Pertussis-Toxin, Pertussis-Toxoid, Clostridium-Exotoxin oder Clostridium-Toxoid oder aus Pflanzen (Typ I RIPs wie Saporin oder Gelonin oder Typ II RIPs wie Ricin oder die A-Kette von Typ II RIPs oder eine den Typ II RIPs homologe A-Kette der Typ I RIPs). Ebenfalls sind hierbei andere, radioaktive, zytotoxisch oder zytostatisch wirkende, nierdermolekulare Moleküle ("small molecules") und andere als die oben beschriebenen Makromoleküle umfaßt.
Unter dem Begriff "Makromoleküle" werden Moleküle mit einer hohen molekularen Komplexität oder einem hohen Molekulargewicht verstanden. Vorzugsweise sind dies Biomoleküle, wie z.B. Biopolymere, insbesondere Proteine, Oligo- oder Polypeptide, aber auch DNA, RNA, Oligo- oder Polynucleotide, prosthetische Gruppen, Lipide, Oligo- und Polysaccharide, sowie deren Modifikationen, sowie auch synthetische Moleküle. Die Proteine umfassen vorzugsweise auch Fusionsproteine. Der Begriff Peptide oder Proteine umfaßt sowohl natürliche als auch synthetische Peptide oder Proteine. Beispiele für natürliche Proteine umfassen u.a. Antikörper, Antikörperfragmente, Rezeptoren, die an ihre spezifischen Liganden binden, peptidische Liganden, die mit ihren spezifischen Rezeptoren oder Peptiddomänen, die mit spezifischen Substraten, einschließlich Proteinen und Coenzymen, und anderen Peptiden oder Enzymen etc. interagieren. Ebenfalls umfaßt sind hiermit rekombinant hergestellte Formen der vorgenannten Proteine oder Peptide. Natürliche Peptide umfassen entsprechend unter anderem Fragmente der oben beschriebenen Proteine, die mit spezifischen Affinitätsliganden interagieren. Synthetische Proteine oder Peptide umfassen sowohl zur Expression gebrachte Pseudogene oder Fragmente davon, als auch Proteine oder Peptide mit einer zufälligen Aminosäuresequenz.
'Unter dem Begriff "niedermolekulare Moleküle" werden Moleküle verstanden, die von geringerer molekularer Komplexität sind, als die oben definierten Makromoleküle. In der Literatur wird der Begriff "kleine Moleküle" ("small molecules") oder "niedermolekulare Moleküle" ("low molecular weight molecules") nicht einheitlich verwendet. In WO 89/03041 und WO 89/03042 werden Moleküle mit Molekülmassen bis 7000 g/mol als kleine Moleküle beschrieben. Üblicherweise werden jedoch Molekülmassen zwischen 50 und 3000 g/mol, häufiger aber zwischen 75 und 2000 g/mol und meistens im Bereich zwischen 100 und 1000 g/mol angegeben. Beispiele sind dem Fachmann aus den Schriften WO86/02736, WO97/31269, US-A-5928868, US-A-5242902, US-A-5468651, US-A-5547853, US-A-5616562, US-A-5641690, US-A-4956303 und US-A-5928643 bekannt.
Als Beispiele für solche kleinen Moleküle können Oligomere oder auch kleine organische Moleküle angeführt werden, wie Oligopeptide, Oligonucleotide, Kohlenhydrate (Glycoside), Isoprenoide oder Lipidstrukturen. In der Literatur stellt zumeist das Molekulargewicht die Definitionsgrundlage für solche kleinen Moleküle dar.

In einer darüber hinaus bevorzugten Ausführungsform der erfindungsgemäßen Verwendung umfasst die rezeptorbindende und/oder rezeptorerkennende Substanz eine weitere Domaine, die eine immunologische Effektorfunktion induzieren kann.
Der Begriff "immunologische Effektorfunktion" beschreibt im Zusammenhang mit der Erfindung u.a. Effektorfunktionen des Immunsystems, die zu einer Elimination der Rezeptor-tragenden Zelle (Zielzelle) führen. Die Elimination ist vorzugsweise eine Induktion von programmiertem Zelltod (Apoptose), aber gegebenenfalls auch eine nekrotische Elimination von Zielzellen. In vitro Verfahren zur Detektion von Zelltod sind dem Fachmann bekannt (siehe u.a. Dulat et al. (2001)).
Die Induktion solcher immunologischen Effektorfunktionen ist vorzugsweise die Induktion eines Signals, das die Zielzelle für das Immunsystem des Organismus markiert. Vorzugsweise wird die Zielzelle erst durch diese Induktion vom Immunsystem erkannt. Ebenso bevorzugt ist, daß durch diese Induktion die Zelle vom Immunsystem besser erkannt wird. Die Erkennung bzw. bessere Erkennung der Zielzelle durch das Immunsystem ermöglicht die Therapie der Zielzelle.

In einer bevorzugten Ausführungsform ist die induzierte immunologische Effektorfunktion eine zelluläre Effektorfunktion.
Beispiele für zellulär vermittelte immunologische Effektorfunktionen sind die Elimination von Zielzellen durch Effektor-T-Zellen, Monozyten oder Makrophagen.

Insbesondere umfaßt die zelluläre Effektorfunktion MHC-vermittelte und F_{c}-Rezeptor-vermittelte Effektorfunktionen.

In einer alternativen bevorzugten Ausführungsform ist induzierte immunologische Effektorfunktion eine humorale Effektorfunktion.
Beispiele für humorale Effektorfunktionen des Immunsystems sind Antikörpervermittelte Reaktionen oder Reaktionen des Komplementsystems. In diesem Zusammenhang ist die Induktion eine Opsonierung von Zielzellen eine bevorzugte Ausführungsform der Erfindung.

Die Figuren zeigen:

### Figur 1: Potentielle Strukturen der unterschiedlichen Rezeptoren von rViscumin und Ricin (schematisch)

Schematische Darstellung der Rezeptoren von Ricin (Figur 1A) und rViscumin (Figur 1 B). Die Darstellung resultiert aus der Interpretation der in Figur 2 und 3 sowie Figur 4 und 5 zusammengefaßten Ergebnisse aus TLC Overlay Assays. Gal = Galactose, GlcNAc = N-Acetyl-Glucosamin, Glc = Glucose, Cer = Ceramid, Sialic = Sialinsäure. Bei den Rezeptoren von rViscumin handelt es sich um Ganglioside mit endständigen α2-6 verknüpften Sialinsäure-Resten. Die Erkennung von durch Ricin erkannten Strukturen (Figur 1A) findet durch rViscumin nicht statt.

### Figur 2: TLC Test zur Identifizierung von rViscumin spezifischen Gangliosiden in verschiedenen Zellfraktionen

TLC Overlay Bindetests von rViscumin mit neutralen GSL (A) und Gangliosiden (B) von humanen Granulocyten. (A) Reihe a: Chromatogramm von 15 µg neutraler GSL (gefärbt mit Orcinol, Gesamt-Zuckerfärbung); Reihe b: zugehöriger Overlay Assay. (B) Reihe a: Chromatogramm von 15 µg humaner Granulocyten Ganglioside (gefärbt mit Resorcinol, Sialinsäurefärbung); Reihe b: zugehöriger Overlay Assay.

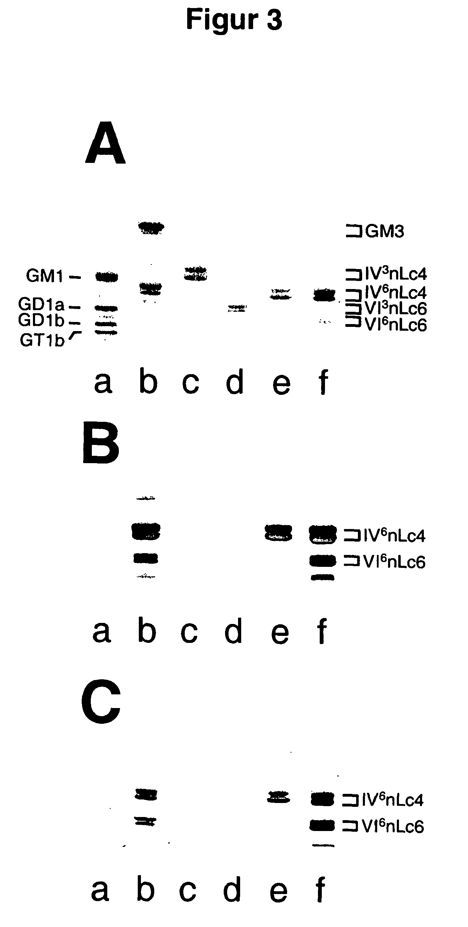

### Figur 3: TLC Test zur Spezifizierung der Carbohydrat Bindespezifität von rViscumin an isolierten Gangliosiden

(A) Resorcinol Färbung (Beispiel 1); (B) anti-IV⁶nLc4Cer Antiserum TLC Overlay Test (Beispiel 1); (C) rViscumin TLC-Overlay Test (Beispiel 2): Alle TLC Assays wurden mit HPLC-gereinigten α2-3- und α2-6-sialylierten Neolacto-Serie Monosialogangliosiden durchgeführt. Der Auftrag ist in allen drei Chromatogrammen identisch: Spuren a: 15 µg Humane Hirn Ganglioside (HBG); Spuren b: 15 µg humane Granulocyten Ganglioside (HGG); Spuren c: 4 µg IV³nLc4Cer (HGG1); Spuren d: 4 µg VI³nLc6Cer (HGG2); Spuren e: 4 µg IV⁶nLc4Cer (HGG3); Spuren f: 8 µg VI⁶nLc6Cer und IV⁶nLc4Cer.

### Figur 4: TLC Test zur Identifizierung von Ricin spezifischen Bindemotiven

(A) Orcinol Färbung und (B) Ricin TLC Overlay Test mit neutralen GSL. Der Auftrag ist in den beiden Chromatogrammen gleich. Spuren a: 10 µg neutrale GSL von humanen Erythrocyten: Spuren b: 15 µg neutrale GSL von humanen Granulocyten; Spuren c: 20 µg neutrale GSL von MDAY-D2 Zellen.

### Figur 5: TLC Test mit Ricin auf Gangliosid-Bindung

(A) Orcinol Färbung und (B) Ricin TLC Overlay Test mit Gangliosiden. Spuren a: 10 µg Humane Hirn Ganglioside (HBG); Spuren b: 8 µg humane Granulocyten Ganglioside (HGG).

### Figur 6: Cytotoxizitätstest mit unterschiedlich sensitiven Zellinien

Beschreibung der biologischen Aktivität des rViscumin: Viabilität von HL-60 Zellen (Punkte), 5637 Zellen (Dreiecke) und CHO-K1 Zellen (offene Kreise) wurde gegen die rViscumin Konzentration aufgetragen. Viabilität wurde mittels colorimetrischer Umsetzung von WST-1 gemessen und als % lebende Zellen im Vergleich zu einer nicht behandelten Kontrolle dargestellt. Die halbmaximale Zytotoxizität, welche dem Wendepunkt der Kurve entspricht wurde als Meßgröße herangezogen. Diese IC₅₀ Werte wurden für HL-60 zu 66 pg/ml und für die 5637 Zellen zu 690 pg/ml berechnet. Die CHO-K1 Zellen sind bis zu einer eingesetzten gemessenen rViscumin-Konzentration von 300 ng/ml als gegenüber rViscumin insensitiv einzustufen.

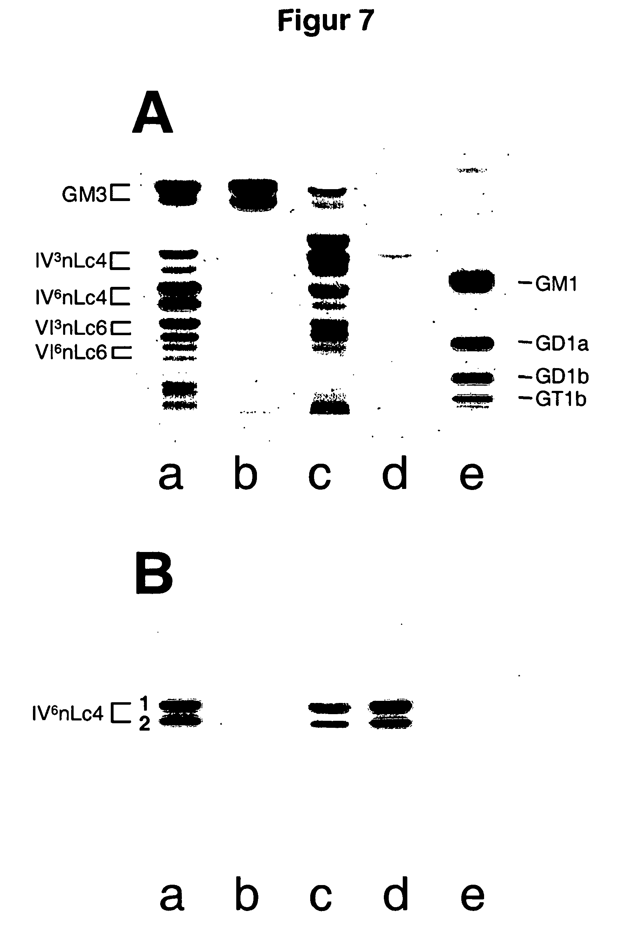

### Figur 7: Semiquantitative Korrelation der Sensitivität gegenüber rViscumin mit Auftreten von rViscumin-spezifischer Gangliosid-Bande

(A) Orcinol Färbung und (B) anti IV⁶nLc4Cer Antiserum TLC Overlay Test mit Gangliosiden aus in vitro propagierten Zellinien.
   (A) Spur a: 7 µg humane Granulocyten Ganglioside (HGG); Spur b: Ganglioside aus 1 x 10⁷ CHO-K1 Zellen; Spur c: Ganglioside aus 4 x 10⁷ 5637-Zellen; Spur d: Ganglioside aus 4 x 10⁷ HL-60 Zellen; Spur e: 10 µg humane Hirn Ganglioside (HBG).
(B) Spur a: 0.134 µg humane Granulocyten Ganglioside (HGG); Spur b: Ganglioside aus 1 x 10⁷ CHO-K1 Zellen; Spur c: Ganglioside aus 1 x 10⁷ 5637-Zellen; Spur d: Ganglioside aus 1 x 10⁷ HL-60 Zellen; Spur e: 10 µg humane Hirn Ganglioside (HBG). Spur a zeigt zur Identifizierung der spezifischen Ganglioside der Neo-Lacto Serie die beiden Positivkontrollen IV⁶nLc4Cer (C24-Fettsäure, Substanz 1) und IV⁶nLc4Cer (C16-Fettsäure, Substanz 2).

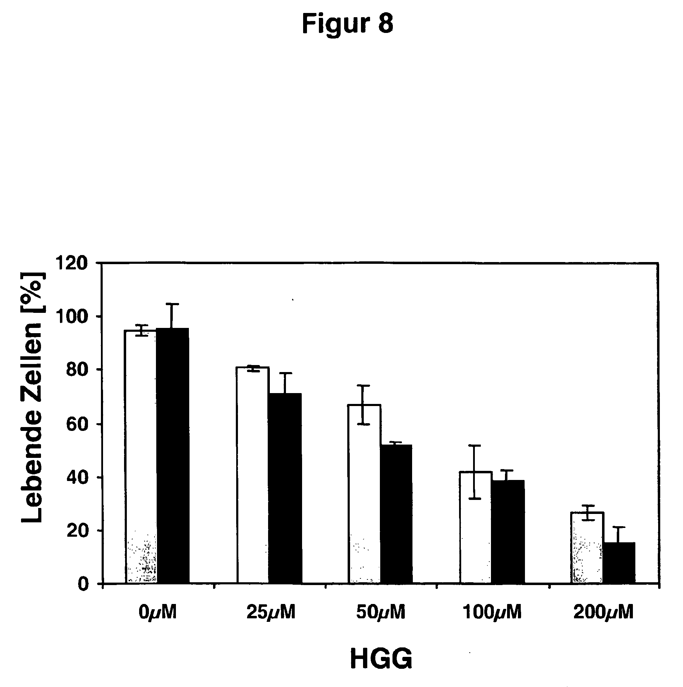

### Figur 8: Sensitivierung der gegenüber rVisucmin insensitiven Zelline CHO-K1 durch Vorinkubation mit spezifischen Gangliosiden.

Steigende Mengen von humanen Granulocyten Gangliosiden (HGG) wurden in mit CHO-K1 Zellen bewachsenen Kavitäten gegeben und dort für 48 inkubiert. Die Zellen wurden entweder mit serumfreiem Medium (hellgraue Balken) oder mit serumhaltigem Medium (dunkle Balken) gewaschen und im Folgenden mit 300 ng/ml rViscumin für weitere 48 h behandelt. Viabilität wurde mit WST-1 gemessen und als % zur unbehandelten Kontrolle (nicht mit Gangliosiden und rViscumin) dargestellt.

### Figur 9: Enzyme-Linked Lectin Assay (ELLA) von rViscumin mit an die Mikrotiterplatte adsorbierten neutralen GSL und Gangliosiden.

Quantitäten der GSL aus verschiedenen Quellen korrespondieren mit den Balken von links nach rechts:
A) Neutrale GSL von humanen Erythrocyten: 10, 5, 2.5, 1.25 und 0 µg
B) Neutrale GSL von humanen Granulocyten: 15, 7.5, 3.75, 1.9 und 0 µg
C) Neutrale GSL von MDAY-D2 Zellen: 20, 10, 5, 2.5 und 0 µg
D) Humane Hirn Ganglioside (HBG): 10, 5, 2.5, 1.25 und 0 µg
E) Humane Granulocyten Ganglioside (HGG): 10, 5, 2.5, 1.25 und 0 µg

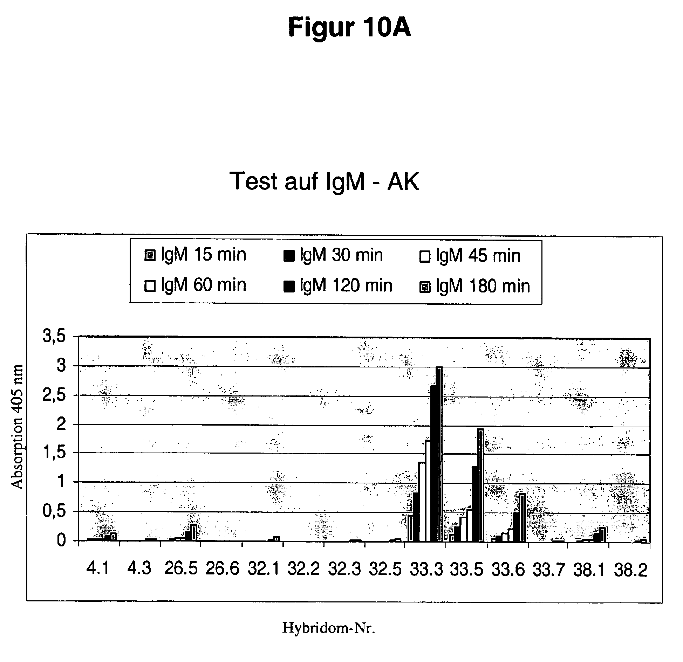

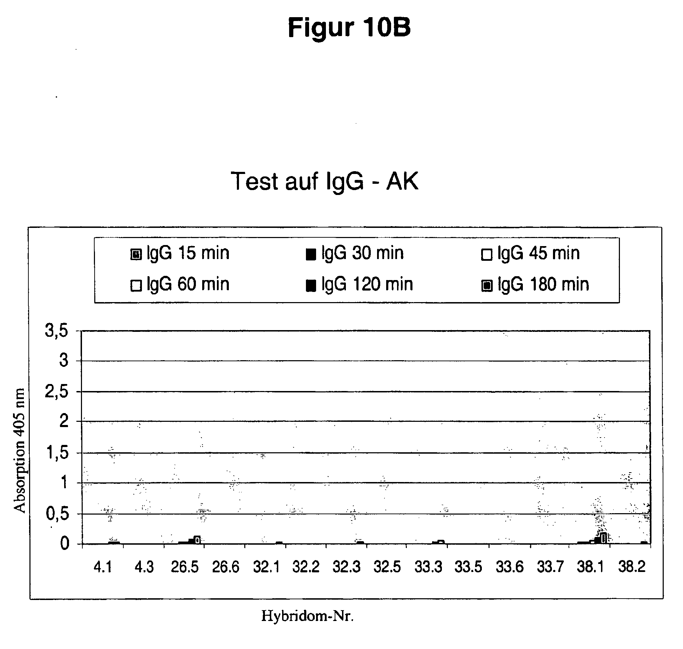

### Figur 10: Identifizierung von spezifischen Antikörpern gegen das zur Immunisierung verwendete Gangliosid-Antigen.

Test verschiedener Hybridom Klone nach Vereinzelung auf Produktion von IgM (A) und IgG (B) im ELISA. Der ELISA wurde wie in Beispiel 8 beschrieben durchgeführt. Die Inkubationszeit der Substratlösung wurde variiert (15 min, 30 min, 45 min, 60 min, 120 min und 180 min). Die Klone 33.3, 33.5 und 33.6 zeigen einen eindeutigen Titer auf IgM (A). Ein Titer auf IgG konnte in keinem der getesteten Klone nachgewiesen werden (B).

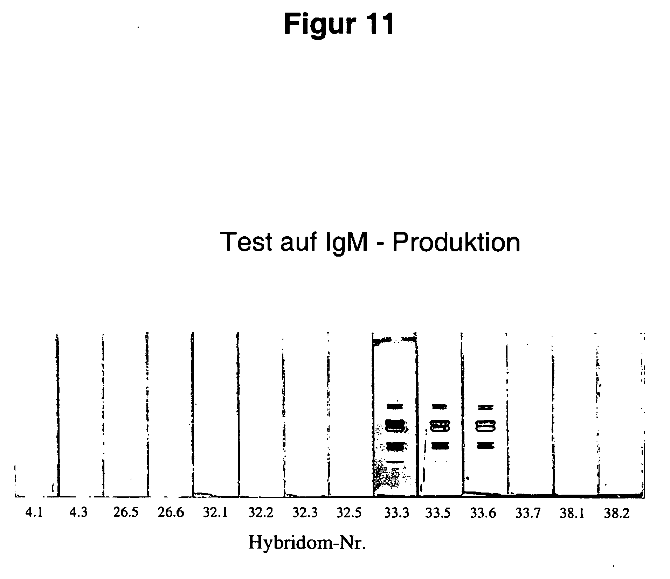

### Figur 11: Charakterisierung von anti-α2-6 sialylierten Neolacto-Typ-Gangliosid-monoklonalen Antikörpern.

TLC Overlay-Assay zur Charakterisierung des Erkennungsmotiv von den mAk-Klonen. Die in bereits in Figur 10 dargestellten Klone wurden, wie in Beispiel 2 beschrieben zum Nachweis von Humanen Ganulozyten Gangliosiden (4 µg/Spur), welche auf den DC-Platten getrennt wurden, auf ihr Erkennungsmotiv hin untersucht. Die Klone 59.33.3, 59.33.5 und 59.33.6 zeigen das zum rViscumin identische Binde- bzw. Erkennungsmotiv.

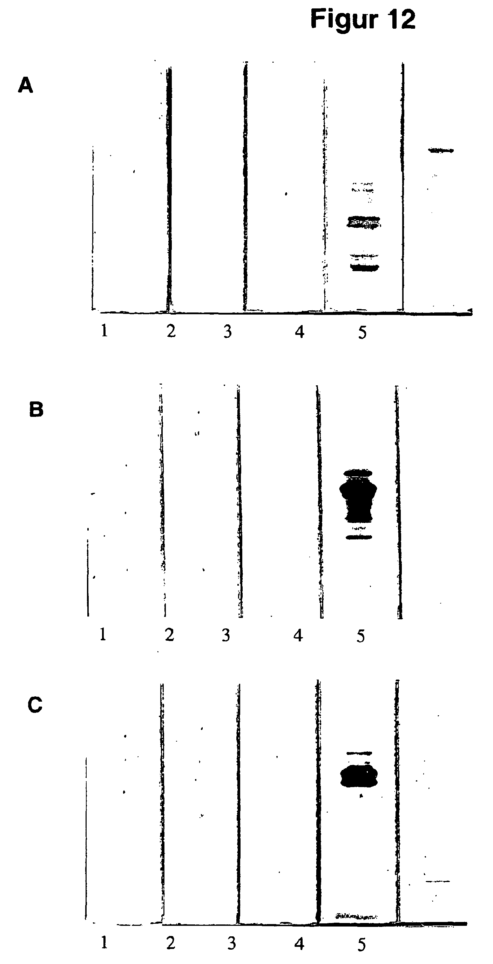

### Figur 12: Weitere Charakterisierung von anti-α2-6 sialylierten Neolacto-Typ-Gangliosid- monoklonalen Antikörpern.

TLC Overlay Assays mit (A) isolierten neutralen GSL der Neolacto-Serie aus humanen Granulozyten (15 µg pro Spur), (B) der Globo-Serie (aus humanen Erythrozyten; 10 µg pro Spur) sowie (C) der Ganglio-Serie (aus MDAY-D2 Zellen; 10 µg pro Spur;). Das experimentelle Vorgehen ist in Beispiel 9 beschrieben. In jeweils den ersten drei Spuren der Figur 12 A bis C wurden die mAk Klone 59.33.3, 59.33.5 und 59.33.6 verwendet. Die Positivkontrollen in den Spuren 4 der Figur 12 A-C reflektieren Reaktionen mit spezifischen Antikörpern gegen die jeweils endständigen Zuckerstrukturen der aufgetragenen neutralen GSL. In den Spuren 5 ist ein polyklonales Antiserum, aus der Ziege (siehe Müthing et al., Glycobiology 12, 485-497) getestet. Eine Kreuzreaktivität der in den Spuren 1 bis 3 getesteten mAk-Klone kann ausgeschlossen werden.

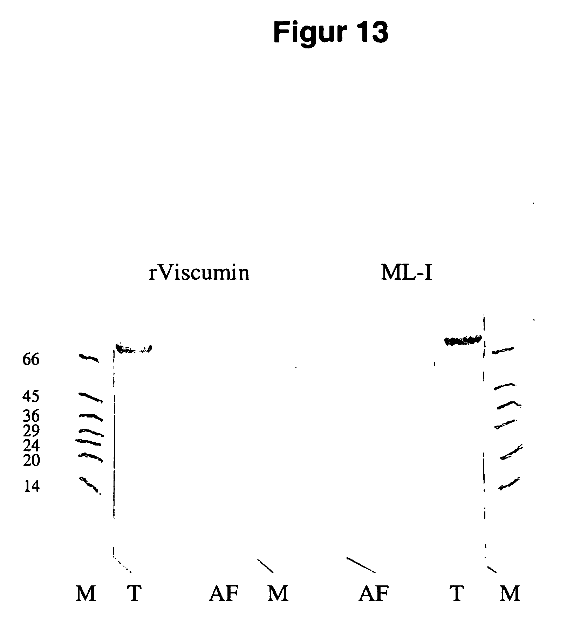

### Figur 13: Nachweis von CD75s-Erkennung von rViscumin auf Glycoproteinen.

Jeweils 1 µg der entsprechenden Proteine, in Spur T: Transferrin, lösliches Protein mit Neu5Aca2-6Galβ1-4GIcNAc-Resten, sowie in Spur AF: Asialofetuin, (Galβ1-4GlcNAc-Resten und Galβ1-3GaINAc-Ser/Thr), wurden im SDS-Gel aufgetragen und anschließend auf eine Nitrocellulose-Membran transferiert. In einem Western Blot Verfahren wurde dann versucht, die CD75s Struktur mit rViscumin (1 µg/ml) und im Anschluß mit dem anti-A-Ketten mAk TA5 und dem mit Alkalischer Phosphatase markierten anti-Maus IgG (siehe Beispiel 2) nachgewiesen. M reflektiert Marker Spuren. Die Molekulargewichte in kDa sind angegeben.

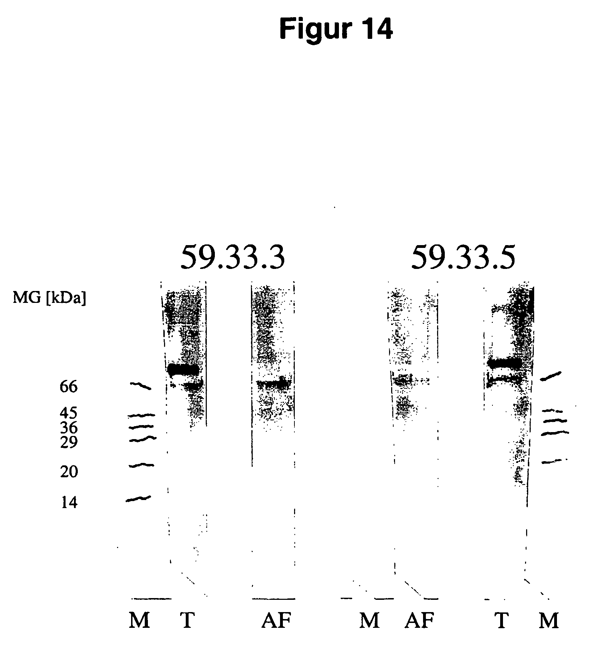

### Figur 14: Nachweis von CD75s-Erkennung von rViscumin auf Glycoproteinen.

Jeweils 1 µg der entsprechenden Proteine, in Spur T: Transferrin, lösliches Protein mit Neu5Aca2-6Galβ1-4GlcNAc-Resten, sowie in Spur AF: Asialofetuin, (Galβ1-4GlcNAc-Resten und Galβ1-3GalNAc-Ser/Thr), wurden im SDS-Gel aufgetragen und anschließend auf eine Nitrocellulose-Membran transferiert. In einem Western Blot Verfahren wurde dann versucht, die CD75s Struktur durch die mAk 59.33.3 sowie 59.33.5, wie in Beispiel 9 beschrieben, nachzuweisen.

**Tabelle 1**

| **Struktur** | **Abk.** | **rViscumin** | **Ricin** |
|---|---|---|---|
| **Ganglioside** | | | |
| Neu5Acα2-3Galβ1-4Glcβ1-1Cer | GM3 | - | - |
| Gatβ1-3GalNAcβ1-4(Neu5Acα2-3)Galβ1-4Glcβ1-1Cer | GM1 | - | (+) |
| Neu5Acα2-3Galβ1-3GalNAcβ1-4(Neu5Acα2-3)Galβ1-4Glcβ1-1Cer | GD1a | - | - |
| Galβ1-3GalNAcβ1-4(Neu5Acα2-8Neu5Acα2-3)Galβ1-4Glcβ1-1Cer | GD1b | - | - |
| Neu5Acα2-3Galβ1-3GalNAcβ1-4(Neu5Acα2-8Neu5Acα2-3)Galβ1-4Glcβ1-1Cer | GT1b | - | - |
| Neu5Acα2-3Galβ1-4GlcNAcβ1-3Galβ1-4Glcβ1-1Cer | IV³nLc4 | - | - |
| Neu5Acα2-6Galβ1-4GlcNAcβ1-3Galβ1-4Glcβ1-1Cer | IV⁶nLc4 | +++++ | - |
| Neu5Acα2-3Galβ1-4GlcNAcβ1-3Galβ1-4GlcNAcβ1-3Galβ1-4Glcβ1-1Cer | VI³nLc6 | - | - |
| Neu5Acα2-6Galβ1-4GlcNAcβ1-3Galβ1-4GlcNAcβ1-3Galβ1-4Glcβ1-1Cer | VI⁶nLc6 | +++++ | - |

| **Neutrale Glykosphingolipide** | | | |
|---|---|---|---|
| Galβ1-4Glcβ1-1Cer | Lc2 | (+) | + |
| Galα1-4Galβ1-4Glcβ1-1Cer | Gb3 | - | (+) |
| GalNAcβ1-3Galα1-4Galβ1-4Glcβ1-1Cer | Gb4 | - | - |
| GalNAcβ1-4Galβ1-4Glcβ1-1Cer | Gg3 | - | - |
| Galβ1-3GalNAcβ1-4Galβ1-4Glcβ1-1Cer | Gg4 | - | +++ |
| Galβ1-4GlcNAcβ1-3Galβ1-4Glcβ1-1Cer | nLc4 | - | +++++ |
| Galβ1-4GlcNAcβ1-3Galβ1-4GlcNAcβ1-3Galβ1-βGlcβ1-1Cer | nLc6 | - | +++++ |
| Galβ1-4(Fucα1-3)GlcNAcβ1-3Galβ1-4GlcNAcβ1-3Galβ1-4Gtcβ1-1Cer | Lewis^{x} | - | - |

Die folgenden Beispiele erläutern die beschriebene Erfindung.

### Beispiel 1: Dünnschichtchromatographie zur Trennung und zum Nachweis von Glykosphingolipiden sowie zu deren spezifischen Nachweis

Neutrale GSL wurden aus humanen Granulozyten, humanen Erythrozyten und MDAY-D2 Zellen und Ganglioside aus humanen Granulocyten und humanem Gehirn gewonnen (Abbildung 2, Abbildung 4, Abbildung 5 und Abbildung 7) oder es wurden HPLC-gereinigte Proben zur Analyse eingesetzt (Abbildung 3). Die jeweiligen Proben wurden auf einer mit Kieselgel beschichteten Dünnschichtchromatographie Platte (HPTLC-Platten, 10 x 10 cm, 0.2 mm Dicke von Merck, Darmstadt # 5633) separiert. Neutrale GSL wurden in Solvens 1 (Chloroform/Methanol/Wasser, 120/70/17 v/v/v) und Ganglioside in Solvens 2 (Chloroform/Methanol/Wasser 120/85/20 v/v/v + 2 mM CaCl₂) getrennt. Neutrale GSL wurden mit Orcinol gefärbt, Ganglioside wurden entweder mit Orcinol oder mit Resorcinol gefärbt (Svennerholm, 1956, 1957). Neutrale GSL von humanen Granulocyten und MDAY-D2 Zellen wie auch Ganglioside von humanen Granulocyten erscheinen auf der DC-Platte als Doppelbanden.
Zur Identifizierung von einzelnen Gangliosiden wurde eine Immun-Färbe Prozedur nach Müthing und Mühlradt (1988) sowie Müthing (1998) mittels spezifischer Antiseren durchgeführt. Terminal α2-6-sialylierte Neolacto-Serie Ganglioside wurden mit einem polyklonalen anti-IV⁶nLc4Cer Antiserum identifiziert. Dazu wurde die Platte zunächst mit Polyisobutylmethacrylat (Röhm, Darmstadt) fixiert. Anschließend wurde die Platte in PBS (20 mM Phosphat, 150 mM NaCl, pH 7.2) + 1 % BSA (Lösung A) geblockt. Im Folgenden wurde das o.g. polyklonale Antiserum in einer 1:1000 Verdünnung in Lösung A eingesetzt und anschließend 3x mit Lösung B (PBS + 0.05% Tween 20) gewaschen. Gebundenes Antiserum wird mit dem Sekundärantiserum Kaninchen Anti Huhn IgY in einer 1:2000 Verdünnung in Lösung A nachgewiesen. Danach wird 3 x mit Lösung B und 1 x mit Glycin Puffer (0.1 M Glycin, 1 mM ZnCl2, 1 mM MgCl2, pH 10.4) gewaschen. Der Nachweis erfolgt mit 0.05% (w/v) 5-Brom-4-Chlor-3-Indolylphosphat in Glycin-Puffer. Die in den verschiedenen TLC-Tests eingesetzten eingehend charakterisierten Kontroll-Ganglioside, welche als Referenzmaterial zur Identifizierung der für rViscumin spezifischen Ganglioside eingesetzt wurden, entstammten verschiedenen Isolierungen aus humanen Blutzellen oder Zellkulturen (Müthing et al., 1994; Müthing und Kemminer, 1996; Schwartz et al., 1985; Müthing und Cacic, 1997).

### Beispiel 2: TLC Test zum spezifischen Nachweis von an neutralen GSL oder Gangliosiden gebundener Carbohydrat-Bindeproteine wie Ricin und rViscumin

Auf einer Dünnschichtchromatographie-Platte getrennte neutrale GSL oder Ganglioside wurden mit PBS-Tween 80 (0.1g/L Tween 80 in PBS) gewaschen. Im Anschluß wurde mit rViscumin oder Ricin Proteinlösung (1 µg/ml in PBS-T80) überschichtet und für 1 h inkubiert. Im Anschluß wurde mit Lösung B (siehe Beispiel 1) gewaschen und dann wurde die Platte für 15 min in Lösung A (siehe Beispiel 1) inkubiert. Daran schloß sich eine Erkennung des gebundenen rViscumin durch den monoklonalen AK TA5 (1µg/ml in Lösung A) beschrieben bei Tonevitzky et al. 1995, bzw. eine Erkennung des gebundenen Ricins durch ein Kaninchen-Anti-Ricin Antiserum von Sigma #R-1254 (1:200 Verdünnung in Lösung A) an. Die Detektion der gebundenen Antikörper erfolgte wie in Beispiel 1 beschrieben mit den jeweils benötigten Zweitantikörpern gegen Maus bzw. Kaninchen Immunglobulin.

### Beispiel 3: Vergleich der Sensitivität verschiedener Zellinien auf rViscumin

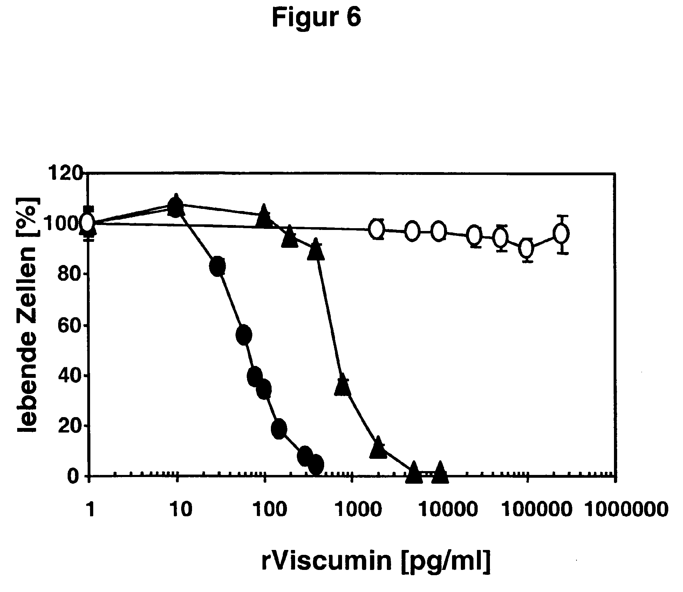

Kultivierung: CHO-K1 Zellen (ATCC CCL-81) und HL-60 Zellen (ATCC CCL-240) wurden in einem 1:1 Mix von DMEM und Ham's F12 Medium gezogen. Die humane Blasenkarzinomzellinie 5637 (ATCC HTB-9) wurde in RPMI 1640 kultiviert. Eine Kultivierung der Zellen erfolgte unter Standardbedingungen wie bei Möckel et al. 1997 beschrieben. Die Sensitivität der Zellinien auf rViscumin wurde in einem in vitro Cytotoxizitätstest ermittelt. 8 x 10³ 5637, 1 x 10⁴ CHO-K1 und 1.8 x 10⁴ HL-60 jeweils pro Kavität wurden in 96-Loch-Platten (Nunc) ausplattiert und mit steigenden Konzentrationen an rViscumin in einem finalen Volumen von 100 µl für 72 h bei 37 °C unter 10% CO₂ Atmosphäre inkubiert. Alle Messungen wurden in Sechsfachbestimmung durchgeführt. Nach 72 h wurde zum Nachweis von lebenden Zellen 10 µl WST-1 zupipettiert (Ishiyama et al., 1993; Möckel et al., 1997). Die Zellen wurden mit dem Farbstoffsalz 4 h bei 37 °C inkubiert und im Anschluß bei 450 nm im Thermomax von Molecular Devices vermessen. Die Menge an Farbstoff ist dabei direkt proportional zu der Anzahl lebender Zellen. In Abbildung 6 sind die Viabilitätskurven der verschiedenen Zellpopulationen gegen die steigenden rViscumin-Konzentrationen aufgetragen.
Die Zellinie HL-60 (Punkte) stellt eine humane promyeloische Zellinie dar, die mit einem IC₅₀ Wert von 66 pg/ml sehr sensitiv auf rViscumin reagiert. In der Blasenkarzinomzellinie 5637 (Dreiecke) wurde ein IC₅₀ Wert von 690 pg/ml gemessen, die Hamsterzellinie CHO K1 (offene Kreise) zeigte bis zu einer getesteten Konzentration von 300 ng/ml keine Sensitivität gegenüber rViscumin. In Fig. 7 konnte die Sensitivität der verschiedenen Zellinien gegenüber rViscumin mit dem Auftreten des potentiellen Rezeptors korreliert werden. In Fig. 7 A ist ein Orcinol-gefärbtes Dünnschichtchromatogramm der Gesamtgangliosidfraktionen der jeweiligen verwendeten Zellinien dargestellt. Fig. 7 B zeigt den spezifischen immunologischen Nachweis der potentielllen rViscumin-Rezeptoren mit einem Anti-Neu5Acα2-6Galβ1-4GlcNAc-R Antiserum (Overlay-Assay). In den Spuren b, c und d der Fig. 7 B sind jeweils Gangliosidmengen von gleichen Zellzahlen von CHO-K1, 5637 und HL-60 Zellen aufgetragen. Die bezogen auf rViscumin insensitiven CHO-K1 Zellen zeigen in Spur b in Fig. 7 B keine spezifische Bande auf Höhe des potentiellen Rezeptors, während die sensibelste Zellinie (HL-60) bei gleicher aufgetragener Zellzahl die stärkste Doppelbande aufweist.
Die in den TLC Overlay Assays beobachteten unterschiedlichen Spezifitäten der untersuchten Lektine erlauben Rückschlüsse auf das Bindungsverhalten von Ricin und Mistellektin (insbesondere rViscumin) an deren Rezeptoren in vivo bzw. in Zellkulturen. In den Beispielen ist beschrieben, daß die CHO-K1 Zellinie gegenüber einer getesteten rViscumin-Konzentration von bis zu 300 ng/ml insensitiv ist (siehe Figur 6). Durch ein Einbringen der für rViscumin spezifischen Ganglioside in die Membranen der CHO-K1 Zellen (Neu5Ac α2-6 Gal-Strukturen), wurden diese gegenüber rViscumin sehr insensitiven Zellen hingegen sensibilisiert (siehe Figur 8).

### Beispiel 4: Sensitivierung von Zellinien durch Vorinkubation mit spezifischen Gangliosiden (siehe Figur 8)

CHO-K1 Zellen wurden mit einer Zelldichte von 5 x 10³ Zellen pro Kavität in 96-Loch Platten ausgesät. Die Zellen wurden für 24 h in serumfreiem X-Vivo Kulturmedium (Biowhittaker) bei 37 °C kultiviert. Anschließend wurde das Medium verworfen und durch X-Vivo Kulturmedium enthaltend 0 (Kontrolle), 25, 50, 100 und 200 µM einer Humanen Granulocyten Gangliosid-Fraktion ersetzt. Zum Einbau der Ganglioside in die Membran der CHO-K1 Zellen wurden die Zellen für 48 h bei 37 °C unter Zellkultur-Bedingungen gehalten. Die Zellen wurden im Anschluß entweder mit serumfreiem X-Vivo oder mit 5% FCS haltigem X-Vivo extensiv gewaschen. Die Zellen wurden im Anschluß mit rViscumin (300 ng/ml) für 48 h behandelt. Die Viabilität der Zellen wurde mittels WST-1 Färbung quantifiziert und mit der nichtbehandelten Kontrolle korreliert (ohne Zugabe von rViscumin und Gangliosiden). Die Proben wurden in Triplikaten angesetzt mit einem Endvolumen von 100 µl pro Kavität.

### Beispiel 5: Reinigung von Gangliosiden von in vitro propagierten Zellen und Korrelation von Auftreten spezifischer Ganglioside in Zellinien und Sensitivität gegenüber rViscumin

Darstellung von Zellmasse zur anschließenden Isolierung von GSL bzw Gangliosiden: CHO-K1 Zellen (ATCC CCL-81) und HL-60 Zellen (ATCC CCL-240) wurden in einem 1:1 Mix von DMEM und Ham's F12 Medium gezogen. Die Zellen wurden im Beisein von 50 mg/L Gentamycin und 2.5 mg/L Amphotericin B kultiviert. Eine Kultivierung von 5637 Zellen erfolgte in 175 cm² Flaschen. HL-60 und CHO-K1 Zellen wurden in einer 1 L Spinnerflasche kultiviert wie unter Duvar et al. (1996) bzw. Müthing et al. (1996b) beschrieben. Die physiologischen Einstellungen waren: 37°C, pH 7.2, Begasung mit Sauerstoff, Rührgeschwindigkeit: 40 rpm. Nach Erreichen der finalen Zelldichten wurden die Zellen geerntet, 2 x mit PBS gewaschen und anschließend einer Chloroform/Methanol-Extraktion unterzogen (siehe Beispiel 1). Die humane Blasenkarzinomzellinie 5637 (ATCC HTB-9) wurde in RPMI 1640 kultiviert. Die Ganglioside wurden nach Standard-Prozeduren unter Nutzung von Anionentauscher Chromatographie an DEAE-Sepharose CL-6B isoliert, wie sie bei Ledeen und Yu (1982) Müthing et al. (1987) oder Duvar et al. (1997) beschrieben sind. Zur Verseifung der Phospholipide wurden die Ganglioside für 1 h bei 37°C in 1 N NaOH inkubiert. Anschließend wurde die Gangliosid-Fraktion mit Essigsäure neutralisiert und dialysiert. Ein weiterer Reinigungsschritt schloß sich über Adsorptionschromatographie an latrobeads 6RS-8060 (Macherey & Nagel, Düren, Germany) an (Ueno et al., 1978). Bei der sich anschließenden Trennung der Gangliosidfraktion aus den Zelllysaten wurde immer von einer etwa einheitlichen Zellzahl ausgegangen. Damit war eine semiquantitative Aussage zum Vorhandensein des spezifischen Gangliosids möglich. Der Nachweis erfolgte wie in Beispiel 1 beschrieben immunologisch mit einem polyklonalen anti-IV⁶nLc4Cer Antiserum.

### Beispiel 6: Reinigung von terminal α2-3- und α2-6-sialylierten Gangliosiden der Neolacto-Serie

Eine mit Fractogel EMD TMAE-650-S (MERCK, Darmstadt) gefüllte Säule wird mit den Gangliosiden aus humanen Granulocyten (HGG) beladen. An TMAE gebundene Ganglioside werden mit einem linearen Gradienten an Ammoniumacetat eluiert, gepoolt und entsalzt. Es wurden vier Fraktionen mit terminal α2-3 sialylierten (HGG1 und HGG2) und terminal α2-6 sialylierten Neolacto-Typ Monosialogangliosiden (HGG3 und HGG4) erhalten. Alle Ganglioside wurden auf Dünnschicht Chromatogrammen als Doppelbanden separiert: Die Unterschiede sind in der Ceramid-Einheit begründet, die im Falle der oberen Bande eine C24 Fettsäure, im Falle der unteren Bande eine C16 Fettsäure verestert hat. Die Strukturen aller über die Methode darstellbaren Ganglioside wurden bereits charakterisiert (Müthing et al., 1993; Müthing et al., 1996, Metelmann et al., 2000). Die gereinigten Ganglioside sind in Abbildung 3 aufgetrennt worden und wurden zur genauen Spezifizierung der Carbohydrat-Bindespezifität von rViscumin herangezogen.

### Beispiel 7: Enzyme-linked lectin assay (ELLA)

Der ELLA wurde in mit den entsprechenden neutralen GSL oder Gangliosiden beschichteten Platten (NUNC MaxiSorp F96, Wiesbaden) bei RT beschichtet. Die Kavitäten wurden mit den entsprechenden GSL in 100 µl Methanol befüllt. Ausgehend von einer GSL Stammlösung wurden 1:2 Verdünnungen hergestellt. Dies beinhaltet folgende GSL: Neutrale GSL von humanen Erythrocyten: 10, 5, 2.5 und 1.25 µg; Neutrale GSL von humanen Granulocyten: 15, 7.5, 3.75 und 1.9 µg; Neutrale GSL von MDAY-D2 Zellen: 20, 10, 5, und 2.5 µg; Humane Hirn Ganglioside (HBG): 10, 5, 2.5 und 1.25 µg; Humane Granulocyten Ganglioside (HGG): 10, 5, 2.5 und 1.25 µg. Anschließend wurde das Methanol in einer trockenen Atmosphäre verdampft (45 min bei 37 °C). Sämtliche weiteren Schritte wurden wie im Beispiel 2 beschriebenen TLC-Overlay Assay durchgeführt. Alle Inkubationen erfolgten in einem Volumen von 100 µl. Die GSL beschichteten Platten wurden für 15 min mit PBS-Tween 80® (0.01mg/ml) inkubiert und im Anschluß mit 1 µg rViscumin (gelöst in PBS-Tween 80) für 1 h inkubiert. Danach wurden die Platten mit PBS-Tween 20® (0.01mg/ml) gewaschen und dann 15 min in PBS inkubiert. Anschließend werden die Proben mit murinem anti ML-1 A monoklonalen Antikörper TA5 (1 µg/ml in PBS) für 1 h inkubiert. Nach erneutem 3 maligen Waschen mit PBS-Tween 20® wird mit einem anti-Maus IgG (Alkalische Phosphatase gekoppelt) in einer 1:2000 Verdünnung in PBS inkubiert (1 h). Nach erneutem 3 maligen Waschen mit PBS-Tween 20® erfolgt der kolorimetrische Nachweis mit der Umsetzung des Substrates Di Natrium-4-nitrophenylphosphat x 6 H₂O (16 mM in 0.1 M Glycin-Puffer, pH 10.4). Die Platte wurde nach 20 min Inkubation bei 25 °C bei 405 nm im Mikrotiterplatten Reader vermessen. Wider Erwarten konnte eine Bindung von rViscumin nur an solche Glycosphingolipide (GSL) gefunden werden, die endständig an der Neu5Ac eine α2-6 gebundene Galactose an GSL der Neolacto-Serie exponierten (HGG Fraktion aus humanen Granulocyten). Alle anderen neutralen GSL aus verschiedenen Quellen (humane Granulozyten und Erythrocyten, MDAY-D2 Zellen) jeweils mit endständigen Galactose-Resten sowie Humanhirnganglioside der Ganglio-Serie zeigten selbst in der höchsten eingesetzten Konzentration keine Bindung.

### Beispiel 8: Herstellung von monoklonalen Antikörpern gegen α2-6 sialylierte Neolacto-Typ Ganglioside.

Die α2-6 sialylierten Neolacto-Typ Ganglioside wurden aus Leukozyten ("buffy Coat" aus Vollblut) isoliert. Die Reinigung der Ganglioside erfolgte wie in Beispiel 5 beschrieben. Im weiteren Verlauf dienten diese α2-6 sialylierten Neolacto-Typ Ganglioside als Antigene zur Immunisierung von 2 x 3 Mäusen. Da die Antigene allein aufgrund ihrer zu geringen Größe nicht immunogen wirken, werden sie den Mäusen mit sog. Haptenen verabreicht. Dabei wurden zwei Verfahren parallel durchgeführt. In Verfahren 1 wurde Methyl-BSA als Hapten in einer Konzentration von 1 mg/ml eingesetzt. Die drei Mäuse bekamen unterschiedliche Mengen an Antigen (5, 10 und 20 Mikrogramm). Die Mäuse wurden in bekannten Abständen immunisiert und insgesamt weitere 2 mal im Abstand von 10 Tagen geboostert. Im Verfahren 2 wurde ein Gemisch aus Liposomen und Lipopolysaccharid (LPS) als Hapten eingesetzt. Auch hier wurden den drei Mäusen unterschiedliche Mengen an Antigen (5, 10 und 20 Mikrogramm) jeweils an drei Applikationstagen im Abstand von 10 Tagen verabreicht.
Aus den insgesamt sechs Mäusen, die mittels zweier Verfahren immunisiert wurden, konnten in Blut von drei Mäusen positive Titer gegen das Antigen ermittelt werden. Die Ansätze mit dem Hapten Methyl-BSA waren negativ. Die Ansätze mit dem Hapten Lipopolysaccharid in Liposomen waren unabhängig von der gegebenen Antigendosis alle positiv. Eine Maus wurde getötet und die Milzzellen zur Fusion herangezogen. Die Überstände der Fusionsansätze wurden im DC-Overlay Assay (wie in Beispiel 1 und 2 beschrieben) und im ELISA untersucht. Dabei wurde die Mikrotiterplatte wie in Beispiel 7 beschrieben mit 1 µg HGG Gangliosiden (Human granulocyte gangliosides) in 50 µl Methanol beschichtet. 50 µl der entsprechenden Plasmaproben bzw. Überstände der Fusionsansätze wurden unverdünnt auf die beschichteten Platten gegeben und für 60 min bei 25 °C inkubiert. Anschließend wurde 3 x mit PBS-T gewaschen. Ein Nachweis von IgM bzw. IgG erfolgte mit einem anti Maus-IgG bzw anti-Maus IgM Antikörper, der mit Alkalischer Phosphatase markiert war. Eine Umsetzung des Substrates p-NPP wurde wie in Beispiel 7 beschrieben durchgeführt. Fusionsansätze mit positivem Titer wurden nachfolgend auf Klonebene vereinzelt. In Figur 10A ist ein Nachweis auf drei positive IgM Klone gezeigt (59.33.3, 59.33.5 sowie 59.33.6). Alle Klone wurden auch auf die Produktion von IgG untersucht, zeigten jedoch auf IgG keine positive Reaktion (Figur 10B).

### Beispiel 9: Charakterisierung von anti-α2-6 sialylierten Neolacto-Typ-Gangliosid monoklonalen Antikörpern

α2-6 sialylierten Neolacto-Typ-Ganglioside tragen endständig immer ein Neu5Acα2-6-Gal. Ein weiteres auch im Zuge dieser Erfindung beschriebenes Motiv umfasst die Gangliosidstruktur Neu5Acα2-6-Galβ1-4GlcNAc-R; wobei "R" für Rest steht. Dieses Motiv ist in der Literatur als CD75s beschrieben (Schwartz-Albiez (2000), J. Biol. Regul. Homeost. Agents 14, 284-285). In Figur 11 sind DC-Overlay Assays mit verschiedenen IgM-Klonen, welche wie in Beispiel 8 beschrieben im ELISA getestet wurden, dargestellt. Mittels Dünnschichtchromatographie wurden hier die Ganglioside aus humanen Granulocyten (HGG) aufgetrennt. Der Assay wird wie in Beispiel 2 beschrieben durchgeführt. Es wurden hier 4 µg HGG getrennt. Der Nachweis von an die Platte gebundenen anti-CD75s mAk erfolgte wie in Beispiel 8 beschrieben. Die Klone 59.33.3, 59.33.5 sowie 59.33.6 zeigten auch in diesem Test ein positives Signal. Alle drei Klone erkannten die gleichen Ganglioside (IV⁶nLC4 sowie IV⁶nLC6) und hatten somit das identische Bindeverhalten wie rViscumin (Figur 2Bb). Eine eventuell zu erwartende Kreuzreaktivität der drei Antikörper gegen den Stammbereich der Zuckerstrukturen wurde unter Verwendung von isolierten neutralen GSL aus entweder der Neolacto-Serie aus humanen Granulozyten (15 µg pro Spur; Figur 12A), der Globo-Serie (aus humanen Erythrozyten; 10 µg pro Spur; in Figur 12B) sowie der Ganglio-Serie (aus MDAY-D2 Zellen; 10 µg pro Spur; in Figur 12C) konnte experimentell ausgeschlossen werden. Das experimentelle Vorgehen war identisch mit oben beschriebenen. In jeweils den ersten drei Spuren der Figur 12 A bis C konnten unter Verwendung der mAk Klone 59.33.3, 59.33.5 und 59.33.6 keine Signale detektiert werden. Das Fehlen der endständig α2-6 verknüpften Sialinsäure führt in allen drei Fällen dazu, daß ein spezifisches Signal ausbleibt. Die Positivkontrollen in den Spuren 4 der Figur 12 A-C reflektieren Reaktionen mit spezifischen Antikörpern gegen die jeweils endständigen Zuckerstrukturen der aufgetragenen neutralen GSL. In den Spuren 5 ist ein polyklonales Antiserum, aus der Ziege (siehe Müthing (2002) Glycobiology 12, 485-497) mitgeführt worden. Dieses Antiserum, welches ursprünglich gegen α2-6 sialylierten Neolacto-Typ-Ganglioside hergestellt wurde, zeigt gegen viele der hier aufgetragenen Antigene eine Kreuzreaktivität, was insbesondere bei einer Verwendung dieses Antiserums in einer Untersuchung von Patientenmaterial zu falsch-positiven Signalen führen könnte. Um so wichtiger sind die Ergebnisse mit den in den Spuren 1 bis 3 dargestellten monoklonalen Antikörpern 59.33.3, 59.33.5 sowie 59.33.6 einzuschätzen, bei denen solche ungewünschten Kreuzreaktivitäten nicht auftauchten. Der mAk-Klon 59.33.3 wurde am 20. Dezember 2002 unter der Zugriffsnummer DSM ACC2580 bei der DSMZ in Braunschweig gemäß Budapester Vertrag hinterlegt.

### Beispiel 10: Nachweis von CD75s auf Glycoproteinen

Die monoklonalen Antikörper 59.33.3 und 59.33.5 (Figur 14) sowie rViscumin (Figur 13) selbst wurden auf ihre Verwendbarkeit zur Detektion von CD75s Motiven auf Glycoproteinen hin untersucht. Hier wurden jeweils 1 µg der entsprechenden Proteine, in Spur T: Transferrin, lösliches Protein mit NeuSAca2-6Galβ1-4GlcNAc-Resten, sowie in Spur AF: Asialofetuin, (Galβ1-4GIcNAc-Resten und Galβ1-3GalNAc-Ser/Thr), im SDS-Gel aufgetragen und anschließend auf eine Nitrocellulose transferiert. In einem Western Blot Verfahren wurde dann versucht, die CD75s Struktur entweder mit rViscumin (1 µg/ml) und im Anschluß mit dem anti-A-Ketten mAk TA5 und dem mit Alkalischer Phosphatase markierten anti Maus IgG (siehe Beispiel 2) nachgewiesen. Im Falle der Prüfung der Bindung/Erkennung des CD75s Epitopes durch die mAk 59.33.3 sowie 59.33.5 wurde nach Transfer der Proteine auf die Nitrocellulose wie in Beispiel 9 beschrieben verfahren. Das Transferrin wird eindeutig von dem mAks 59.33.3 und 59.33.5 sowie von rViscumin erkannt.
Die beiden getesteten Antikörperklone 59.33.3 und 59.33.5 erkennen CD75s Motive auf dem Glycoprotein Transferrin als Epitope.

### Literatur:

Agapov, I.I., Tonevitsky, A.G., Shamshiev, A.T., Phol, E., Pohl, P., Palmer, R.A., Kirpichnikov, M.P. (1997): The role of structural domains in RIP II Toxin model membrane binding. FEBS Lett., 402, 91-93
Andre S., Unverzagt,C., Kojima,S., Dong,X., Fink,C., Kayser,K., Gabius,H.J. (1997) Neoglycoproteins with the synthetic complex biantennary nonasaccharide or its alpha 2,3/ alpha2/6-sialylated derivatives: their preparation, assessment of their ligand properties for purified lectins, for tumor cells in vitro, and in tissue sections, and their biodistribution in tumor-bearing mice. Bioconjug Chem 8, 845-855
Barbieri,L., Battelli,M.G. and Stirpe,F. (1993) Ribosome-inactivating proteins from plants. Biochim. Biophys. Acta, **1154,** 237-282.
Beuth J, Ko HL, Gabius HJ, Pulverer G. (1991) Influence of treatment with the immunomodulatory effective dose of the beta-galactoside-specific lectin from mistletoe on tumor colonization in BALB/c-mice for two experimental model systems. In Vivo; 5(1): 29-32.
Beuth J, Ko HL, Gabius HJ, Burrichter H, Oette K, Pulverer G. (1992) Behavior of lymphocyte subsets and expression of activation markers in response to immunotherapy with galactoside-specific lectin from mistletoe in breast cancer patients. Clin Investig.; 70(8): 658-61.
Beuth J, Ko HL, Tunggal L, Geisel J, Pulverer G. (1993) [Comparative studies on the immunoactive action of galactoside-specific mistletoe lectin. Pure substance compared to the standardized extract]. Arzneimittelforschung.; 43(2):166-9. German language.
Bocci V. (1993) Mistletoe (viscum album) lectins as cytokine inducers and immunoadjuvant in tumor therapy. J Biol Regul Homeost Agents; 7(1): 1-6.
Bonk, *Biopsie und Operationspräparat;* Kompendium für Ärzte und Studenten
Critchley, D.R. Streuli, C.H., Kellie, S., Ansell, S., and Patel., B. (1982): Characterisation of the cholera toxin receptor on Balb/c 3T3 cells as a ganglioside similar to, or identical with, ganglioside GM1. No evidence for galactoproteins with receptor activity. Biochem J., 204, 209-219
Debray,H., Montreuil,J. and Franz,H. (1994) Fine sugar specificity of the mistletoe (Viscum album) lectin I. Glycoconjugate J., 11, 550-557.
Dulat, HJ., von Grumbkow, C, Baars, W., Schroder, N., Wonigeit, K., Schwinzer, R. (2001) Down-regulation of human alloimmune responses by genetically engineered expression of CD95 ligand on stimulatory and target cells. Eur J Immunol., 7, 2217-26
Duvar,S., Müthing,J., Mohr,H. and Lehmann,J. (1996) Scale up cultivation of primary human umbilical vein endothelial cells on microcarriers from spinner vessels to bioreactor fermentation. Cytotechnology, **21**, 61-72.
Duvar,S., Peter-Katalini,J., Hanisch,F.-G. and Müthing, J. (1997) Isolation and structural characterization of glycosphingolipids of *in vitro* propagated bovine aortic endothelial cells. Glycobiology, **7,** 1099-1109.
Eck,J., Langer,M., Möckel,B., Baur,A., Rothe,M., Zinke,H. and Lentzen,H. (1999a) Cloning of the mistletoe lectin gene and characterization of the recombinant A-chain. Eur. J. Biochem., **264,** 775-784.
Eck,J., Langer,M., Möckel,B., Witthohn,K., Zinke,H. and Lentzen,H. (1999b) Characterization of recombinant and plant-derived mistletoe lectin and their B-chain. Eur. J. Biochem., **265,** 788-797.
Endo Y, Oka T, Tsurugi K, Franz H. (1989) The mechanism of action of the cytotoxic lectin from Phoradendron californicum: the RNA N-glycosidase activity of the protein. FEBS Lett.; 248(1-2): 115-8.
Franz H, Haustein B, Luther P, Kuropka U, Kindt A. (1977) Isolation and characterization of mistletoe extracts (Viscum album L.). I. Affinity chromatography of mistletoe extracts on immobilized plasma proteins. Acta Biol Med Ger.; 36(1): 113-7.
Franz,H. (1986) Mistletoe lectin and their A and B chains. Oncology, **43,** 23-24.
Frantz,M., Jung,M.L., Riberau-Gayon,G., and Anton,R. (2000) Modulation of mistletoe lectin (Viscum album L.) Ictins cytotoxicity by carbohydrates and serum glycoproteins. Arzneimittelf./Drug Res. 50,471-478
Gabius HJ, Walzel H, Joshi SS, Kruip J, Kojima S, Gerke V, Kratzin H, Gabius S. (1992) The immunomodulatory beta-galactoside-specific lectin from mistletoe: partial sequence analysis, cell and tissue binding, and impact on intracellular biosignalling of monocytic leukemia cells. Anticancer Res.; 12(3): 669-75.
Gabius HJ, Gabius S, Joshi SS, Koch B, Schroeder M, Manzke WM, Westerhausen M. (1994) From ill-defined extracts to the immunomodulatory lectin: will there be a reason for oncological application of mistletoe? Planta Med., 60(1): 2-7.
Gabius HJ und Gabius S; Die Misteltherapie auf dem naturwissenschaftlichen Prüfstand. PZ., 1994; 139, 9-16.
Galanina,O.E., Kaltner,H., Khraltsova,L.S., Bovin,N.V. and Gabius,H.-J. (1997) Further refinement of the description of the ligand-binding mistletoe lectin, a plant agglutinin with immunomodulatory potency. J. Mol. Recogn., **10**, 139-147.
Ganguly C and Das S. (1994) Plant lectins as inhibitors of tumour growth and modulators of host immune response. Chemotherapy; 40(4): 272-8.
Gilleron,M., Siebert,H.-C., Kaltner,H., von der Lieth,C.-W., Kozar,T., Halkes,K.M., Korchagina,E.Y., Bovin,N.V., Gabius,H.-J. and Vliegenthart,J. (1998) Conformer selection and differential restriction of ligand mobility. Eur. J. Biochem., **252**, 416-427.
Gottstein, C., Schon, G., Tawadros, S., Kube, D., Wargalla-Plate, U.C., Hansmann, M.L., Wacker, H.H., Berthold, F., Diehl, V., Engert, A. (1994): Antidisialoganglioside ricin A-chain immunotoxins show potent antitumor effects in vitro and in a disseminated human neuroblastoma severe immunodeficiency mouse model. Cancer Res. 54, 6186-6193
Gupta,D., Kaltner,H., Dong,X., Gabius,H.-J. and Brewer,C.F. (1996) Comparative cross-linking activities of lactose-specific plant and animal lectins and a natural lactose-binding immunoglobulin G fraction from human serum with asoalofetuin. Glycobiology, **6**, 843-849.
Hajto T. Immunomodulatory effects of iscador: a Viscum album preparation. Oncology, 1986; 43 Suppl 1: 51-65.
Hajto T, Hostanska K, Gabius HJ. (1989) Modulatory potency of the beta-galactoside-specific lectin from mistletoe extract (Iscador) on the host defense system in vivo in rabbits and patients. Cancer Res.; 49(17): 4803-8.
Hajto T, Hostanska K, Frei K, Rordorf C, Gabius HJ. (1990) Increased secretion of tumor necrosis factors alpha, interleukin 1, and interleukin 6 by human mononuclear cells exposed to beta-galactoside-specific lectin from clinically applied mistletoe extract. Cancer Res.; 50(11): 3322-6.
Hakomori,S.-I., Handa,K., Iwabuchi,K., Yamamura,S. and Prinetti,A. (1998) New insights in glycosphingolipid function: "glycosignaling domain", a cell surface assembly of glycosphingolipids with signal transducer molecules, involved in cell adhesion coupled with signaling. Glycobiology, **8**, xi-xix.
Hanasaki, K., Powell, L.D., Varki, A. (1995) Binding of human plasma sialoglycoproteins by the B cell-specific lectin CD22. Selective recognition of immunoglobulin M and haptoglobin. J Biol Chem. 270(13), 7543-50.
Heiny BM, Beuth J. (1994) Mistletoe extract standardized for the galactoside-specific lectin (ML-1) induces beta-endorphin release and immunopotentiation in breast cancer patients. Anticancer Res.; 14(3B): 1339-42.
Hermann T., Patel DJ. (2000) Adaptive recognition by nucleic acid aptamers. Science; 287 (5454), 820-5.
Hooper,N.M. (1999) Detergent-insoluble glycosphingolipid/cholesterol-rich membrane domains, lipid rafts and caeolae (Review) Mol. Membr. Biol., **16,** 145-156.
Ishiyama,M., Shiga,M., Sasamoto,K., Mizoguchi,M. and He,P. (1993) A new sulfonated tetrazolium salt that produces a highly water-soluble formazan dye. Chem. Pharm. Bull., **41**, 1118-1122.
Kaltner, H., Lips, K.S., Reuter, G., Lippert, S., Sinowatz, F., Gabius, H.J. (1997): Quantitation and histochemical localisation of galectin-1 and galectin-1-reactive glycoconjugates in fetal development of bovine organs. Histol. Histopathol, 12, 945-960
Klein CA, Schmidt-Kittler O, Schardt JA, Pantel K, Speicher MR, Riethmuller G. (1999) Comparative genomic hybridization, loss of heterozygosity, and DNA sequence analysis of single cells. Proc Natl Acad Sci U S A; 96 (8), 4494-9
Kremer et al. (1999), *Chirurgische Operationslehren,* Georg Thieme Verlag, Stuttgart/New York, Band 4, S. 186; Band 7, 2, S. 370; Band E, Seiten 146 bis 147 und Band 5, S. 238
Langer, M., Möckel, B., Eck, J., Zinke, H., Lentzen, H. (2000) Both biochemical activities of rViscumin are prerequisite for its cytotoxic action. Proceedings of the American Association for Cancer Research, 41, 655
Ledeen,R.W. and Yu,R.K. (1982) Gangliosides: structure, isolation and analysis. Methods Enzymol., **83**, 139-191.
Lee,R.T., Gabius,H.-J. and Lee,Y.C. (1992) Ligand binding characteristics of the major mistletoe lectin. J. Biol. Chem., **267**, 23722-23727.
Lee,R.T., Gabius,H.-J. and Lee,Y.C. (1994) The sugar combining area of the galactose-specific toxic lectin of mistletoe extends beyond the terminal sugar residue: comparison with a homologous toxic lectin, ricin. Carbohydr. Res., **254**, 269-276.
Lee, J., Sundaram, S., Sharper, N.L., Raju, T.S., and Stanley, P. (2001): CHO cells may express six {beta}4Galactosyltransferases. Consequences of the loss of funcitonal {beta}4GalT-1, {beta}4GalT-6 or both in CHO glycosyltion mutants. J. Biol. Chem. Feb. 2nd
Magnani, J. L., Nilsson, B., Brockhaus, M., Zopf, D., Steplewski, Z., Koprowski, H. and Ginsburg, V. (1982) A monoclonal antibody-defined antigen associated with gastrointestinal cancer is a ganglioside containing sialylated lacto-*N*-fucopentaose II, J. Biol. Chem. 257, 14365-14369.
Malte (1998), *Angiologie in Klinik und Praxis,* Georg Thieme Verlag, S. 258
Mannel DN, Becker H, Gundt A, Kist A, Franz H. (1991) Induction of tumor necrosis factor expression by a lectin from Viscum album. Cancer Immunol Immunother.; 33(3): 177-82.
Metelmann,W., Müthing,J. and Peter-Katalini,J. (2000) Nano-electrospray ionization quadrupole time-of-flight tandem mass spectrometry analysis of a ganglioside mixture from human granulocytes. Rapid Commun. Mass Spectrom., **14**, 543-550.
Möckel,B., Schwarz,T., Zinke,H., Eck,J., Langer,M. and Lentzen,H. (1997) Effects of mistletoe lectin I on human blood cell lines and peripheral blood cells: cytotoxicity, apoptosis and induction of cytokines. Drug Res., **47**, 1145-1151.
Müthing, J., Egge, H., Kniep, B., and Mühlradt, P. F. (1987) Structural characterization of gangliosides from murine T lymphocytes, Eur. J. Biochem. 163, 407-416.
Müthing, J. and Mühlradt, P. F. (1988) Detection of gangliosides of the G_{M1b} type on high-performance thin-layer chromatography plates by immunostaining after neuraminidase treatment, Anal. Biochem. **173,** 10-17.
Müthing, J., Unland, F., Heitmann, D., Orlich, M., Hanisch, F.-G., Peter-Katalinic, J., Knäuper, V., Tschesche, H., Keim, S., Schauer, R. and Lehmann, J. (1993) Different binding capacities of influenza A and Sendai viruses to gangliosides from human granulocytes, Glycoconjugate J. **10**, 120-126.
Müthing,J., Maurer,U., Sostari,K., Neumann,U., Brandt,H., Duvar,S., Peter-Katalinic J. and Weber-Schürholz,S. (1994) Different distributions of glycosphingolipids in mouse and rabbit skeletal muscle demonstrated by biochemical and immunohistolical analyses. J. Biochem., **115,** 248-256.
Müthing,J. and Kemminer,S.E. (1996) Nondestructive detection of neutral glycosphingolipids with lipophilic anionic fluorochromes and their employment for preparative high-performance thin-layer chromatography. Anal. Biochem., **238,** 195-202.
Müthing,J., Spanbroek,R., Peter-Katalini,J., Hanisch,F.-G., Hanski,C., Hasegawa,A., Unland,F., Lehmann,J., Tschesche,H. and Egge,H. (1996a) Isolation and structural characterization of fucosylated gangliosides with linear poly-N-acetyllactosaminyl chains from human granulocytes. Glycobiology, **6**, 147-156.
Müthing,J., Duvar,S., Nerger,S., Büntemeyer,H. and Lehmann,J. (1996b) Microcarrier cultivation of bovine aortic endothelial cells in spinner vessels and a membrane-stirred bioreactor. Cytotechnology, **18**, 193-206.
Müthing,J. and Cacic, M. (1997) Glycosphingolipid expression in human skeletal and heart muscle assessed by immunostaining thin-layer chromatography. Glycoconjugate J., 14, 19-28.
Müthing,J. (1998) TLC in structure and recognition studies of glycosphingolipids. In Hounsell,E.F. (ed.), Methods in Molecular Biology, Vol. 76: Glycoanalysis Protocols. Humana Press Inc., Totowa, NJ, pp. 183-195.
Mülhardt, C. (2000) Der Experimentator: Molekularbiologie, 2. Auflage, Spektrum Akad. Verlag
Niethard und Pfeil (1997) *Orthopädie,* 3. Auflage, Hippokrates Verlag Stuttgart.
Olsnes,S., Stirpe,F., Sandvig,K. and Phil,A. (1982) Isolation and characterization of viscumin, a toxic lectin from Viscum album L. (mistletoe). J. Biol. Chem., **257**, 13263-13270.
Pan CL, Yuki N, Koga M, Chiang MC, Hsieh ST. Acute sensory ataxic neuropathy associated with monospecific anti-GD1b IgG antibody. Neurology 2001, 9;57(7):1316-8
Pohl, P., Saparow, S.M., Pohl, E.E., Evtodienko, V.Y., Agapov, I.I., and Tonevitsky, A.G. (1998a): Dehydration of model membranes induced by lectins from Ricinus communis and Viscum album. Biophys. J., 75, 2868-2876
Pohl, P., Antonenko, Y.N., Evtodienko, V.Y., Pohl, E.E., Saparow, S.M., Agapov, I.I., and Tonevitsky, A.G. (1998b): Membrane fusion mediated by ricin and viscumin. Biochim Biophys Acta, 1371, 11-16
Pichlmayr und Löhlein (1991), *Chirurgische Therapie,* Springer Verlag, S. 225, 130, 89, 93, 116, 546 und 547
Radsak,K., Schwarzmann,G. and Wiegandt,H. (1982) Studies on cell association of exogenously added sialo-glycolipids. Hoppe-Seyter's Z. Physiol. Chem., **363**, 263-272.
Rehm, H. (2000) Der Experimentator: Proteinbiochemie / Proteomics, 3 Auflage, Spektrum Akad. Verlag
Rutenber, E., Ready, M, and Robertus, J.D., (1987), Structure and evolution of ricin B-chain. Nature, 326, 624-626
Samal, A.B., Gabius, H.J., Timoshenko, A.V. (1995) Galactose-specific lectin from Viscum album as a mediator of aggregation and priming of human platlets. Anticancer Res. 15, 361-367
Saqr, H.E., Pearl, D.K., and Yates, A.J. (1993) A review and predictive model of ganglioside uptake by biological membranes. J. Neurochem., 61, 395-411
Schwartz,R., Kniep,B., Müthing,J. and Mühlradt,P.F. (1985) Glycoconjugates of murine tumor lines with different metastatic capacities. II. Diversity of glycolipid composition, Int. J. Cancer, **36**, 601-607.
Simons,K. and lkonen,E. (1997) Functional rafts in cell membranes. Nature, **387**, 569-572.
Stoffel,B., Kramer,K., Mayer,H., and Beuth,J. (1997): Immunomodulating efficacy of combined administration of galactoside-specific lectin standardized mistletoe extract and sodium selenits in BALB/c-mice. Anticancer Res 17, 1893-1896
Svennerholm,L. (1956) The quantitative estimation of cerebrosides in nervous tissue. J. Neurochem.,**1**, 42-53.
Svennerholm,L. (1957) Quantitative estimation of sialic acids. Biochim. Biophys. Acta., **24,** 604-611.
Svennerholm,L. (1963) Chromatographic separation of human brain gangliosides, J. Neurochem., **10**, 613-623.
Thomson, T.A., Hayes, M.M.,Spinelli, J.J., Hilland, E., Sawrenko, C., Phillips, D., Dupuis, B. und Parker, R.L. (2001) Her-2/neu in breast cancer: interobserver variability and performance in immunohistochemistry with 4 antibodies compared with fluorescent in situ hybridisation. Mod. Pathol. 14, 1079-1086
Tonevitsky, A.G., Zhukova, O.S., Mirimanova, N.V., Omelyanenko, V.G., Timofeeva, N.V., and Bergelson, L.D. (1990) Effect of gangliosides on binding, internalisation and cytotoxic activity of ricin. FEBS Lett., 264, 249-252
Tonevitsky,A.G., Rakhmanova,V.A., Agapov, I.I., Shamshiev,A.T., Usacheva,E.A., Prokoph'ev,S.A., Denisenko,O.N., Alekseev,Y. and Pfüller,U. (1995) The interactions of anti-MLI monoclonal antibodies with isoform of the lectin from *Viscum album.* Immunol. Lett., **44**, 31-34.
Tur MK, Sasse S, Stocker M, Djabelkhir K, Huhn M, Matthey B, Gottstein C, Pfitzner T, Engert A, Barth S. An anti-GD2 single chain Fv selected by phage display and fused to Pseudomonas exotoxin A develops specific cytotoxic activity against neuroblastoma derived cell lines. Int J Mol Med. 2001 8(5):579-84.
Turpin, E., Goussault, Y., Lis, H., and Sharon, N. (1984): Nature of the receptor sites for galactosyl-specific lectins on human lymphocytes. Exp. Cell Res., 152, 486-492
Ueno,K., Ando,S. and Yu,R.K. (1978) Gangliosides of human, cat, and rabbit spinal cords and cord myelin. J. Lipid Res., **19**, 863-871.
Usui, H. and Hakomori, S. (1994): Evaluation of ricin A chain-containing immunotoxins directed against glycolipid and glycoprotein on mouse lymphoma cells. Acta Med. Okayama 48, 305-309
Utsumi, T., Aizono, Y., and Funatsu, G. (1987): Receptor-mediated interaction of ricin with the lipid bilayer of ganglioside GM1-liposomes. FEBS Lett., 216, 99-103
Vang, O., Pii Larsen, K., And Bog-Hansen, T.C. (1986) A new quantitative highly specific assay for lectinbinding activity. In: Lectins. Biology, Biochemistry, Clinical Biochenistry Vol. 5; Eds.: Bog-Hansen, T.C., and van Driessche, E. pp637-644, W. de Gruyter, Berlin, N.Y.
Voet, D., Voet, JG., (1994) Wiley-VCH Verlag
Wu, A.M., Chin, L.K., Franz, H., Pfüller, U., Herp, A. (1992): Carbohydrate Specificity of the receptor sites of mistletoe toxin lectin I. Biochim Biophys. Acta, 1117, 232-234
Wu,A.M., Song,S.-C., Hwang,P.-Y., Wu,J.H. and Pfüller,U. (1995a) Interaction of mistletoe toxic lectin-I with sialoglycoproteins. Biochem. Biophys. Res. Commun., **214**, 396-402.
Wu,A.M., Watkins,W.M., Chen,C-P., Song,S-C., Chow L-P., and Lin,J-Y (1995b) Native and/or asialo-Tamm-Horsfall glycoproteins sd(a+) are important receptors for triticum vulgaris (wheatgerm) agglutinin and for three toxic lectins (abrin-a ricin and mistletoe toxic lectin I) FEBS Lett., 371, 32-34

## Patentansprüche

1. In vitro Verfahren zur Bestimmung der Responsivität eines Individuums für Mistellektin oder für (eine) Mistellektin-Einzelketten, umfassend den Schritt des spezifischen quantitativen und/oder qualitativen Nachweises eines membranständigen Rezeptors, wobei der Rezeptor durch eine endständige N-Acetyl-Neuraminsäure (Neu5Ac), die über eine glykosidische α2-6-Verknüpfung mit einer Galactose (Gal) verbunden ist, **gekennzeichnet** ist.

2. Verfahren nach Anspruch 1, wobei der Rezeptor ein Gangliosid umfaßt, das nach der α2-6-gebundenen N-Acetyl-Neuraminsäure an Galactose mindestens ein N-Acetyl-Glucosamin (GIcNAc) umfaßt.

3. Verfahren nach Anspruch 1 oder 2, wobei der Rezeptor ein Gangliosid der Struktur Neu5Acα2-6-Gal-[Gal/GlcNAc]_{X}-Cer umfaßt.

4. Verfahren nach einem der Ansprüche 1 bis 3, wobei der Rezeptor ein Gangliosid der Struktur Neu5Acα2-6-[Galβ1-4GlcNAcβ1-3]ₓGalβ1-4Glcβ1-1 Cer umfasst.

5. Verfahren nach einem der Ansprüche 1 bis 4, wobei der Rezeptor ein Gangliosid der Struktur Neu5Acα2-6Galβ1-4GlcNAcβ1-3Galβ1-4GIcβ1-1Cer oder ein Gangliosid der Strukur Neu5Acα2-6Galβ1-4GIcNAcβ1-3Galβ1-4GlcNAcβ1-3Galβ1-4GIcβ1-1Cer umfaßt.

6. Verfahren nach einem der Ansprüche 1 bis 5, wobei der Rezeptor zellmembranständig ist.

7. Verfahren nach einem der Ansprüche 1 bis 6, wobei das Mistellektin ein rekombinantes Mistellektin/rViscumin ist.

8. Verfahren nach Anspruch 7, wobei das rekombinante Mistellektin eine Aminosäuresequenz umfaßt, die durch ein Polynucleotid wie in SEQ ID Nr: 1, SEQ ID Nr: 3 oder SEQ ID Nr: 5 dargestellt, codiert wird.

9. Verfahren nach Anspruch 7 oder 8, wobei das rekombinante Mistellektin ein Polypeptid mit einer Aminosäuresequenz wie in SEQ ID Nr: 2, SEQ ID Nr: 4 oder SEQ ID Nr: 6 dargestellt oder ein funktionelles Fragment hiervon umfaßt.

10. Diagnostische Zusammensetzung, umfassend eine Substanz, die spezifisch einen Rezeptor, wie in den Ansprüchen 1 bis 9 definiert, erkennt oder bindet und ausgewählt ist aus der Gruppe von Antikörpern, Antikörperderivaten, Antikörperfragmenten und Aptameren, wobei
(i) die Substanz detektierbar markiert ist; oder
(ii) der Antikörper ein monoklonaler Antikörper ist, der aus einer Hybridomzellinie gewonnen werden kann, die am 20. Dezember 2002 bei der DSMZ Braunschweig unter Zugriffsnummer DSM ACC2580 hinterlegt wurde.

11. Verwendung einer Substanz, die spezifisch einen Rezeptor, wie in den Ansprüchen 1 bis 9 definiert, erkennt oder bindet und ausgewählt ist aus der Gruppe von Antikörpern, Antikörperderivaten, Antikörperfragmenten und Aptameren, zur Herstellung eines Diagnostikums zum Nachweis eines funktionellen Mistellektin-Rezeptors.

12. Verwendung nach Anspruch 11, wobei die Substanz detektierbar markiert ist.

13. Verwendung nach Anspruch 11 oder 12, wobei der Antikörper ein monoklonaler Antikörper ist.

14. Verwendung nach Anspruch 13, wobei der monoklonale Antikörper aus einer Hybridomzellinie gewonnen werden kann, die am 20. Dezember 2002 bei der DSMZ Braunschweig unter Zugriffsnummer DSM ACC2580 hinterlegt wurde.

15. Verwendung nach einem der Ansprüche 11 bis 14, wobei das Diagnostikum zur Detektion des Rezeptors, wie definiert in einem der Ansprüche 1 bis 8, auf Zellen verwendet wird.

16. Verwendung nach Anspruch 15, wobei die Zellen Tumorzellen sind.

17. Verwendung nach Anspruch 15 oder 16, wobei die Zellen aus Biopsiematerial stammen.

18. Verwendung nach Anspruch 17 oder 18, wobei die Zellen aus Blutproben isoliert werden.

## Claims

1. An in vitro method for the determination of the responsiveness of an individual to mistletoe lectin or to (a) mistletoe lectin single chains, comprising the step of the specific quantitative and/or qualitative detection of a membrane-bound receptor, wherein the receptor is **characterised by** a terminal N-acetyl neuraminic acid (Neu5Ac) which is linked to a galactose (Gal) by a glycosidic α2-6 bond.

2. The method according to claim 1, wherein the receptor comprises a ganglioside which after the N-acetyl neuraminic acid bound to galactose via an α 2-6 bond at least comprises an N-acetyl glucosamine (GlcNAc).

3. The method according to claim 1 or 2, wherein the receptor comprises a ganglioside of the structure Neu5Acα 2-6-Gal-[Gal/GlcNAc]ₓ-Cer.

4. The method of any one of claims 1 to 3, wherein the receptor comprises a ganglioside with the structure Neu5Acα 2-6-[Galβ1-4GlcNAcβ1-3]ₓ Galβl-4Glcβ1-1Cer.

5. The method of any one of claims 1 to 4, wherein the receptor comprises a ganglioside with the structure Neu5Acα2-6Galβ1-4GlcNAcβ1-3Galβ1-4Glcβ1-1Cer or a ganglioside with the structure Neu5Acα2-6Galβ1-4GlcNAcβ1-3Galβ1-4GlcNAcβ1-3Galβ1-4Glcβ1-1Cer.

6. The method of any one of claims 1 to 5, wherein the receptor is cell membrane-bound.

7. The method of any one of claims 1 to 6, wherein the mistletoe lectin is a recombinant mistletoe lectin/rViscumin.

8. The method of claim 7, wherein the recombinant mistletoe lectin comprises an amino acid sequence which is encoded by a polynucleotide as shown in SEQ ID NO: 1, SEQ ID NO: 3 or SEQ ID NO: 5.

9. The method according to Claim 7 or 8, wherein the recombinant mistletoe lectin is a polypeptide with an amino acid sequence as shown in SEQ ID NO: 2, SEQ ID NO: 4 or SEQ ID NO: 6 or comprises a functional fragment thereof.

10. A diagnostic composition, comprising a substance which specifically recognises or binds to a receptor as defined in Claims 1 to 9 and is selected from the group of antibodies, antibody derivatives, antibody fragments, aptamers, wherein
(i) the substance is detectably labelled; or
(ii) the antibody is a monoclonal antibody, which can be recovered from a hybridoma cell line, which was deposited on December 20, 2002 with the DSMZ Braunschweig under the accession number DSM ACC2580.

11. Use of a substance which specifically binds to a receptor or recognises a receptor according to any one of claims 1 to 9, and is selected from the group consisting of antibodies, antibody derivatives, antibody fragments and aptamers for the preparation of a diagnostic agent for the detection of a functional mistletoe lectin receptor.

12. The use according to Claim 11, wherein the substance is detectably labelled.

13. The use according to Claim 11 or 12, wherein the antibody is a monocional antibody.

14. The use according to Claim 13, wherein the monocional antibody can be recovered from a hybridoma cell line deposited on December 20, 2002 at the DSMZ Braunschweig under the accession number DSM ACC2580.

15. The use according to any one of Claim 11 to 14, wherein the diagnostic agent is used for the detection of the receptor, as defined in any one of Claims 1 to 8, on cells.

16. The use according to Claim 15, wherein the cells are tumour cells.

17. The use according to Claim 15 or 16, wherein the cells originate from biopsy material.

18. The use according to Claim 17 or 18, wherein the cells are isolated from blood samples.

## Revendications

1. Procédé de détermination *in vitro* de la capacité d'un individu à répondre à la mistellectine ou à une/des chaîne(s) individuelles de mistellectine, comprenant l'étape consistant en une détection quantitative ou qualitative spécifique d'un récepteur membranaire, le récepteur étant **caractérisé par** un acide N-acétyl-neuraminique terminal (Neu5Ac) qui est lié par une liaison α2-6 glycosidique à un galactose (Gal).

2. Procédé selon la revendication 1, dans lequel le récepteur comprend un ganglioside comprenant après l'acide N-acétyl-neuraminique à liaison α2-6 au galactose, au moins une N-acétyl-glucosamine (GIcNAc).

3. Procédé selon la revendication 1 ou 2, dans lequel le récepteur comprend un ganglioside de structure Neu5Acα2-6-Gal-[Gal/GlcNAc]ₓ-Cer.

4. Procédé selon l'une quelconque des revendications 1 à 3, dans lequel le récepteur comprend un ganglioside de structure Neu5Acα2-6-[Galβ1-4GlcNAcβ1-3]ₓ-Galβ1-4Glcβ1-1Cer.

5. Procédé selon l'une quelconque des revendications 1 à 4, dans lequel le récepteur comprend un ganglioside de structure Neu5Acα2-6-Galβ1-4GlcNAcβ1-3Galβ1-4Glcβ1-1Cer ou un ganglioside de structure Neu5Acα2-6-Galβ1-4GlcNAcβ1-3Galβ1-4GlcNacβ1-3Galβ1-4Glcβ1-1Cer.

6. Procédé selon l'une quelconque des revendications 1 à 5, dans lequel le récepteur est un récepteur de membrane cellulaire.

7. Procédé selon l'une quelconque des revendications 1 à 6, dans lequel la mistellectine est une mistellectine/viscumine recombinante.

8. Procédé selon la revendication 7 dans lequel la mistellectine recombinante comprend une séquence d'acides aminés qui est codée par un polynucléotide tel que représenté dans SEQ ID N° : 1, SEQ ID N° : 3 ou SEQ ID N° : 5.

9. Procédé selon la revendication 7 ou 8, dans lequel la mistellectine recombinante comprend un polypeptide présentant une séquence d'acides aminés telle que dans SEQ ID N° : 2, SEQ ID N° : 4 ou SEQ ID N° : 6 ou un fragment fonctionnel de celles-ci.

10. Composition diagnostique comprenant une substance qui reconnaît ou se lie spécifiquement à un récepteur tel que défini dans les revendications 1 à 9, et qui est choisie dans le groupe des anticorps, des dérivés d'anticorps, des fragments d'anticorps et des aptamères, sachant que
(i) la substance est marquée de façon détectable ; ou
(ii) l'anticorps est un anticorps monoclonal qui peut être obtenu à partir d'une lignée cellulaire d'hybridome qui a été déposée le 20 décembre 2002 auprès de la DSMZ Braunschweig sous le numéro d'accès DSM ACC2580.

11. Utilisation d'une substance qui reconnaît ou se lie spécifiquement à un récepteur tel que défini dans les revendications 1 à 9, et est choisie dans le groupe des anticorps, dérivés d'anticorps, fragments d'anticorps et aptamères, pour la production d'un diagnostic permettant de détecter un récepteur de la mistellectine fonctionnel.

12. Utilisation selon la revendication 11, dans laquelle la substance est marquée de façon détectable.

13. Utilisation selon la revendication 11 ou 12, dans laquelle l'anticorps est un anticorps monoclonal.

14. Utilisation selon la revendication 13, dans laquelle l'anticorps monoclonal peut être obtenu d'une lignée de cellules d'hybridome, qui a été déposée le 20 décembre 2002 auprès de la DSMZ Braunschweig sous le numéro d'accès DSM ACC2580.

15. Utilisation selon l'une quelconque des revendications 11 à 14, dans laquelle le diagnostic pour la détection du récepteur tel que défini dans l'une quelconque des revendications 1 à 8, est utilisé sur des cellules.

16. Utilisation selon la revendication 15, dans laquelle les cellules sont des cellules tumorales.

17. Utilisation selon la revendication 15 ou 16, dans laquelle les cellules proviennent de matériau de biopsie.

18. Utilisation selon la revendication 17 ou 18, dans laquelle les cellules sont isolées à partir d'échantillons sanguins.
